# EUROPEAN PATENT APPLICATION

(11) **EP 2 918 272 A1**
(43) Date of publication of application: **16.09.2015**
(21) Application number: 14185692.2
(22) Date of filing: 24.10.2008
(51) Int. Cl.: A61K 31/445, A61K 9/127, A61P 35/00

(54) **Methods and pharmaceutical compositions for regulation of G- and/or GC-rich nucleic acid expression**

(30) Priority: 22.11.2007 US 989831 P
(62) Divisional of application: 08860508.4
(71) Applicant: Wu, Sophia Shu Fen, Yonkers, NY 10703 (US); Wu, Rong-Tsun, Taipei 112 (TW)
(72) Inventor: Wu, Sophia Shu Fen, Yonkers, NY 10703 (US); Wu, Rong-Tsun, Taipei 112 (TW)
(74) Representative: Straus, Alexander

(57) **Abstract**

Methods and pharmaceutical compositions for regulations of Guanosine- (G-) and/or Guanosine-cytosine-rich (GC-rich) nucleic acid expressions are provided. The methods include a step of interacting the G- and/or GC-rich region of the nucleic acid with thalidomide, and the pharmaceutical compositions include the thalidomide and a pharmaceutical carrier.

## Description

### CROSS-REFERENCE TO RELATED PATENT APPLICATION

This application claims priority to provisional application No.60/989,831 filed November 22, 2007, the entirety of which is incorporated herein by reference.

Some references, which may include patents, patent applications and various publications, are cited and discussed in the description of this invention. The citation and/or discussion of such references is provided merely to clarify the description of the present invention and is not an admission that any such reference is "prior art" to the invention described herein. All references cited and discussed in this specification are incorporated herein by reference in their entireties and to the same extent as if each reference was individually incorporated by reference.

### FIELD OF THE INVENTION

The present application relates generally to the methods and pharmaceutical compositions for regulating the expression of a nucleic acid. More specifically, the present application relates to the methods and pharmaceutical compositions for regulating the expression of Guanosine- (G-) and/or Guanosine-cytosine-rich (GC-rich) nucleic acid.

### BACKGROUND OF THE INVENTION

Thalidomide is a racemic compound and chemically named 2-(2,6-dioxo-3-piperidinyl)-1H-iso-indole-1,3(2H)-dione. Despite the high risk of teratogenicity, thalidomide is emerging as a drug for treating cancer and inflammatory disease (Franks *et al.,* 2004). Furthermore, with its anti-angiogenic and immunomodulatory activities, thalidomide is also considered as an effective drug for treating refractory multiple myeloma (Singnal *et al.,* 1999). Actually, in addition to the myeloma, thalidomide has been widely tested on various types of cancer such as colorectal cancer (Franks *et al,* 2004), myleodysplastic syndrome, Waldenstrom's macroglobulinemia, myelofibrosis with myeloid metaplasia, brain tumor (Eleutherakis-Papaiakovou *et al.,* 2004), acute myeloid leukemia, non-Hodgkin's lymphoma, lung cancer, breast cancer, neuroendocrine tumors, hepatocellular carcinoma (Kumar *et al.,* 2004), mantle cell lymphoma, pancreatic cancer (Teo *et al.,* 2005), renal cell carcinoma, prostate cancer, Kaposis's sarcoma, melanoma (Richardson *et al.,* 2002) and prolactinoma (Mukdsi *et al.,* 2006). Clinical studies in some immunological disorders, including rheumatoid arthritis, erythema nodosum leprosum, Behcet's syndrome, sarcoidosis, Crohn's disease (Franks *et al.,* 2004), aphthous ulcers (Teo *et al.,* 2005), aphthous stomatitis, lupus erythematosus, prurigo nodularis (Wu *et al.,* 2005), ankylosing spondylitis (Scalapino *et al.,* 2003), chronic heart failure (Gullestad *et al.,* 2005) and graft-versus-host disease (GVHD) after allogeneic bone marrow transplantation and renal transplantation (Richardson et *al.,* 2002; Matthews *et al.,* 2005), further support thalidomide's immunomodulatory properties. The anti-angiogenic activity of thalidomide is also be confirmed in angiogenesis-associated diabetic diseases, such as diabetes retinophathy (Bosco *et al.,* 2003). Although these data hold promise in the treatment of the mentioned diseases and/or disorders, the mechanism of action for thalidomide is still not completely understood. Some reports showed thalidomide treatment could reduce plasma basic fibroblast growth factor (bFGF) level, and a positive response for thalidomide treatment in glioma and multiple myeloma (Fine *et al.,* 2000; Neben *et al.,* 2001; Sato *et al.,* 2002). The changes of bFGF level in serum and/or plasma during therapy imply that bFGF might be the target for thalidomide.

bFGF belongs to the FGF gene family and is a potent autocrine and/or paracrine mitogen that is expressed ubiquitously. bFGF participates in many biological activities including stimulation of mesodermal formation, angiogenesis, smooth muscle cell proliferation and regulation of development of nervous system and eye (Bikfalvi *et al.,* 1997). bFGF is known to be overexpressed in various types of tumors, such as brain tumor, prostate cancer (Eleutherakis-Papaiakovou *et al.,* 2004), prolactinoma (Mukdsi *et al.*, 2006), breast cancer (Fuhrmann-Benzakein *et al.,* 2000), head and neck cancer, soft tissue sarcoma, renal cell carcinoma, colorectal carcinoma, hepatocellular carcinoma, ovarian carcinoma, endometrial carcinoma (Poon *et al.,* 2001), melanoma (Ugurel *et al.,* 2001), lung cancer (Ueno *et al.,* 2001; Iwasaki *et al.,* 2004), Kaposis's sarcoma (Samaniego *et al.,* 1998), pancreatic cancer (Yamanaka *et al.,* 1993), multiple myeloma *(Sezer et al.,* 2001), myelodysplastic syndrome, leukemia (Aguayo *et al.,* 2000), non-Hodgkin's lymphoma (Giles *et al.,* 2004) and bladder cancer (Nguyen *et al.,* 1994). bFGF is also associated with sleep disorder (Okumura *et al.,* 1996), immunological disorders and angiogenesis-associated diseases, such as rheumatoid arthritis, osteoarthritis (Nakashima *et al.,* 1994), Crohn's disease (Di Sabatino *et al.,* 2004), Behcet's disease (Erdem *et al.,* 2005), systemic sclerosis (Lawrence *et al.,* 2006), polyarteritis nodosa (Kikuchi *et al.,* 2005), vernal keratoconjunctivitis (Leonardí *et al.,* 2000), psoriasis (Andrys *et al.,* 2007), proliferative diabetic retinopathy (Boulton *et al.,* 1997), age-related macular degeneration (Frank, 1997), asthma (Hoshino *et al.,* 2001) and pulmonary arterial hypertension (Benisty *et al.,* 2004). It is also reported that neoangiogenesis is also an integral part of the immunopathogenesis of chronic inflammatory diseases such as rheumatoid arthritis, psoriasis and retinopathy (Andrys *et al.,* 2007). The secretion of bFGF is independent of the traditional endoplasmic reticulum (ER) -Golgi pathway (Mignatti *et al.,* 1992). In addition to the secreted form, there existed four nuclear-target-forms of bFGF, which are translated alternatively from upstream inframe CUG codons of an internal ribosome entry site (IRES) -dependent mechanism. The structure of IRES is formed by the G-rich N-terminal of bFGF transcripts (Florkiewicz *et al.,* 1989; Vagner *et al.,* 1995). The low molecular weight bFGF (LMW bFGF) is translated by using the first AUG codon of bFGF transcript, and the high molecular weight bFGFs (HMW bFGFs) translated by using the upstream CUG codons. Although the C-terminal part of LMW and HMW bFGFs are the same, the functions are believed to differ from each other due to the different intracellular distributions and the N-terminal extension of HMW bFGFs (Quarto *et al*., 2005). It has been shown that nuclear accumulation of bFGFs or an increased ratio of high HMW bFGFs to LMW bFGF is an indicator for tumor progression (Fukui *et al.,* 2003). Overexpression of bFGF in cancer cells were also correlated to the advanced tumor stage and poor prognosis of pancreatic cancer (Yamanaka *et al*., 1993).

The expression of bFGF transcript is under the control of G-rich promoter, which might be capable of forming secondary structure, such as G-quadruplexes, which could be targeted by some deoxyribonucleic acid (DNA) binding drugs to interact with and subsequently alter the promoter activity (Hurley *et al.,* 2000). It is reported that kinds of genes have G- and/or GC-rich region, such as vascular endothelial growth factor (VEGF), platelet-derived growth factor-A (PDGF-A), hypoxia-inducible factor-1α (HIF-1α), B-cell CLL/lymphoma 2 (Bcl-2), v-myb myeloblastosis viral oncogene homolog (avian) (c-Myb), v-kit Hardy-Zuckerman 4 feline sarcoma viral oncogene homolog (c-Kit), retinoblastoma (Rb), ret proto-oncogene (Ret), avian myelocytomatosis viral oncogene homolog (c-MYC), Kirsten rat sarcoma-2 viral (v-Ki-ras2) oncogene homolog (KRAS) (Qin *et al.*, 2008), type II tumor necrosis factor (TNF) receptor (Bethea *et al.,* 1997), insulin-like growth factor (IGF-1), IGF-1 receptor, integrin, tetraspains and human telomerase reverse transcriptase (hTERT) (Drucker *et al.,* 2003). Besides the transcriptional regulation by the G-rich promoter, the N-terminal extension of bFGF transcript is also G-rich, which could be functioning to regulate the translation of different isoforms. The G-rich region of ribonucleic acid (RNA) transcript can serve as the targets for some DNA binding drugs, and consequently modulation of expression of isoforms.

The teratogenic activity of thalidomide was proposed to be its binding to both DNA and RNA of fetus whether administrated orally or parenterally, and the binding of the thalidomide glutarimide moiety to DNA might alter the secondary structure of DNA (Bakay *et al.,* 1968; Huang *et al.,* 1990; Huang *et al.,* 1999; Nicholls, 1966). Drucker *et al.* reported that Thalidomide could down-regulate transcripts levels for genes with GC-rich promoter in a relative high concentration over 12,5 µg/ml (Drucker *et al.,* 2003).

In addition, some U.S. patents also disclosed the thalidomide could be used in treating immunological disease and cancer and inhibition of angiogenesis, such as U.S. Pat. No. 6,124,322, U.S. Pat. No. 6,235756, U.S. Pat. No. 6,617354, U.S. Pat. No. 6,914,067, U.S. Pat. No. 7,230,012 and U.S. Pat. No. 7,435,726.

U.S. Pat. No. 6,124,322 entitled "Intravenous form of thalidomide for treating immunological diseases" relates to an aqueous Thalidomide solution which is suitable as a parenteral form of application of thalidomide, particularly as an intravenous form of application. U.S. Pat. No. 6,235756 entitled "Methods and compositions for inhibition of angiogenesis by thalidomide" relates to a method for preventing unwanted angiogenesis, particularly in angiogenesis dependent or associated diseases, by administration of compounds such as thalidomide and related compounds. U.S. Pat. No. 6.423,321 entitled "Cytokine antagonists for the treatment of sensorineural hearing loss" relates to the method for inhibiting the action of TNF and/or IL-1 antagonists for treating hearing loss in a human by administering a TNF antagonist and/or an IL-1 antagonist for reducing the inflammation affecting the auditory apparatus of said human, or for modulating the immune response affecting the auditory apparatus of said human, by administering a therapeutically effective dosage level to said human of a TNF antagonist and/or an IL-1 antagonist. U.S. Pat. No. 6,617354 entitled "Method of stabilizing and potentiating the action of anti-angiogenic substances" relates to the use of anti-angiogenic agents in the cure of cell proliferative disorders including cancer and other disorders caused by uncontrolled angiogenic activity in the body. U.S. Pat. No. 6,914,067 entitled "Compositions and methods for the treatment of colorectal cancer" relates to pharmaceutical compositions comprising thalidomide and irinotecan, to methods of treating colorectal cancer, and to methods of reducing or avoiding adverse effects of irinotecan. U.S. Pat. No. 7,230,012 entitled "Pharmaceutical compositions and dosage forms of thalidomide" relates to the pharmaceutical compositions and dosage forms comprising thalidomide and pharmaceutically acceptable prodrugs, salts, solvates, hydrates, and clathrates thereof. And, U.S. Pat. No. 7,435,726 entitled "Compositions and methods for the treatment of cancer" relates to the pharmaceutical compositions including Thalidomide and an anti-cancer agent, particularly a topoisomerase inhibitor, to methods of treating cancer, and to methods of reducing or avoiding adverse effects associated with anti-cancer agents such as topoisomerase inhibitors.

Even though the thalidomide has been used in treating cancer and immunological disease, and inhibition of angiogenesis, the relevant mechanism of action for thalidomide is still not so clear. Therefore, elucidation of the mechanism of action for thalidomide will be beneficial in the methods and/or pharmaceutical compositions for cancer, immunological disorder, angiogenesis-associated disease.

### BRIEF SUMMARY OF THE INVENTION

In one aspect, the present application relates to a method for regulating bFGF expression. The method includes a step of interacting the G- and/or GC-rich region of the bFGF with thalidomide.

Preferably, the thalidomide has a concentration between 100 µg/ml and 0.01 µg/ml.

Preferably, the thalidomide has a concentration between 10 µg/ml and 0.1 µg/ml.

Preferably, the G- and/or GC-rich region has more than 50% GC content therein.

Preferably, the thalidomide is sustainedly released by a drug delivery technology.

Preferably, the thalidomide is encapsulated.

In another aspect, the present application relates to a pharmaceutical composition for regulating bFGF expression. The pharmaceutical composition includes thalidomide.

In a further aspect, the present application relates to a method for treating a disease associated with an expression of bFGF. The method includes a step of interacting the G- and/or GC-rich region of the bFGF with thalidomide.

Preferably, the disease is a bFGF overexpression-associated disease.

Preferably, the bFGF overexpression-associated disease is one selected from the group consisting of cancer, immunological disorder, angiogenesis-associated disease and sleep disorder

Preferably, the cancer is one selected from the group consisting of brain tumor, prostate cancer, pancreatic cancer, breast cancer, lung cancer, head and neck cancer, bladder cancer, renal cell carcinoma, colorectal carcinoma, hepatocellular carcinoma, ovarian carcinoma, endometrial carcinoma, prolactinoma, melanoma, Kaposis's sarcoma, soft tissue sarcoma, multiple myeloma, myelodysplastic syndrome, non-Hodgkin's lymphoma and leukemia.

Preferably, the immunological disorder is one selected from the group consisting of rheumatoid arthritis, osteoarthritis, Behcet's disease, systemic sclerosis, polyarteritis nodosa, psoriasis, asthma, vernal keratoconjunctivitis and Crohn's disease.

Preferably, the angiogenesis-associated disease is one selected from the group consisting of pulmonary arterial hypertension, rheumatoid arthritis, asthma, psoriasis, proliferative diabetic retinopathy and age-related macular degeneration.

In a further aspect, the present application relates to a pharmaceutical composition for treating a disease associated with an expression of bFGF with G- and/or GC-rich region thereof. The pharmaceutical composition includes thalidomide.

In yet another aspect, the present application relates to a method for regulating expression of a DNA and/or RNA having G- and/or GC-rich region. The method includes a step of interacting the G- and/or GC-rich region of the bFGF with thalidomide having a concentration between 100 µg/ml and 0.01 µg/ml.

Preferably, the DNA and/or RNA having G- and/or GC-rich region is one selected from the group consisting of bFGF, VEGF, PDGF-A, HIF-1α, Bcl-2, c-Myb, c-Kit, Rb, Ret, c-MYC, KRAS, type II TNF receptor, IGF-1, IGF-1 receptor, integrin, tetraspains and hTERT.

Preferably, the thalidomide is sustainedly released by a drug delivery technology.

Preferably, the thalidomide is sustained by an encapsulation.

In yet another aspect, the present application relates to a pharmaceutical composition for regulating expression of a DNA and/or RNA having G- and/or GC-rich region. The pharmaceutical composition includes thalidomide between 100 µg/ml and 0.01 µg/ml.

In yet another aspect, the present application relates to a method for treating a disease associated with an expression of a DNA and/or RNA having G- and/or GC-rich region. The method includes a step of interacting the G- and/or GC-rich region with thalidomide having a concentration between 10 µg/ml and 0.1 µg/ml.

Preferably, the disease is one selected from the group consisting of cancer, immunological disorder, angiogenesis-associated disease and sleep disorder.

In a further aspect, the present application relates to a pharmaceutical composition for treating a disease associated with an expression of a DNA and/or RNA having G- and/or GC-rich region. The pharmaceutical composition includes thalidomide between 100 µg/ml and 0.01 µg/ml.

In yet another aspect, the present application relates to a method for increasing bioavailability of thalidomide to bFGF. The method includes a step of retaining a concentration of the thalidomide by a slow-release technology.

Preferably, the concentration of the thalidomide is retained between 10 and 0.1 µg/ml.

In yet another aspect, the present application relates to a pharmaceutical composition for increasing bio-availability of thalidomide to bFGF. The pharmaceutical composition has thalidomide in a slow-release vehicle.

These and other aspects will become apparent from the following description of the preferred embodiment taken in conjunction with the following drawings, although variations and modifications therein may be affected without departing from the spirit and scope of the novel concepts of the disclosure.

The accompanying drawings illustrate one or more embodiments of the invention and, together with the written description, serve to explain the principles of the invention. Wherever possible, the same reference numbers are used throughout the drawings to refer to the same or like elements of an embodiment.

### BRIEF DESCRIPTION OF THE SEVERAL VIEWS OF THE DRAWING

FIG. 1A shows the effect of thalidomide on bFGF transcript levels of U-87 MG cells. Thalidomide (0∼10 µg/ml) was freshly prepared from the stock solution before being added to the cells for treatment of 3 hr.
FIG. 1B shows the effect of thalidomide on bFGF transcript levels of U-87 MG cells. Thalidomide (0∼10 µg/ml) was freshly prepared from the stock solution before being added to the cells for treatment of 12 hr.
FIG. 1C shows the effect of pre-incubation of Thalidomide in culture medium alone on bFGF transcript levels of U-87 MG cells. Thalidomide (0∼10 µg/ml) was incubated with culture medium alone for 9 hr before being added to the cells for treatment of 3 hr.
FIG. ID shows the effect of Thalidomide on bFGF transcript levels of U-87 MG cells. Liposomal Thalidomide (0∼10 µg/ml) was added to the cells for treatment of 12 or 24 hr.
FIG. 2A shows the effect of Thalidomide on bFGF protein expression levels of U-87 MG cells. Liposomal Thalidomide (0∼10 µg/ml) was added to the cells for treatment of 12 hr, and bFGF protein expression levels were determined by FACS analysis.
FIG. 2B shows the effect of thalidomide on the intracellular distribution of bFGF protein. Free-form or liposomal thalidomide (0.1∼10 µg/ml) was added to the U-87 MG cells for treatment of 12 hr, and bFGF protein distribution was examined by fluorescence microscopy. DNAs were stained with Hoechst 33258 as a nuclear marker. The magnification was 400.
FIG. 2C shows the effect of thalidomide on multiple isoforms of bFGF protein expression. Free-form or liposomal thalidomide (0.1∼10 µg/ml) was added to the U-87 MG cells for treatment of 12 hr, and cellular bFGF content was analyzed by Western blot.
FIG. 3A shows the effect of thalidomide on cell proliferation. Free-form or liposomal Thalidomide (0∼100 µg/ml) was added to the U-87 MG cells for treatment of 72 hr, and the relative cell growth was determined by resazurin assay.
FIG. 5B shows inhibition of anchorage-independent growth of U-87 MG cell by thalidomide. Cells were cultured in soft agar containing free-form or liposomal thalidomide (0∼10 µg/ml). Colonies were photographed 14 days after the start of the relevant experiment.

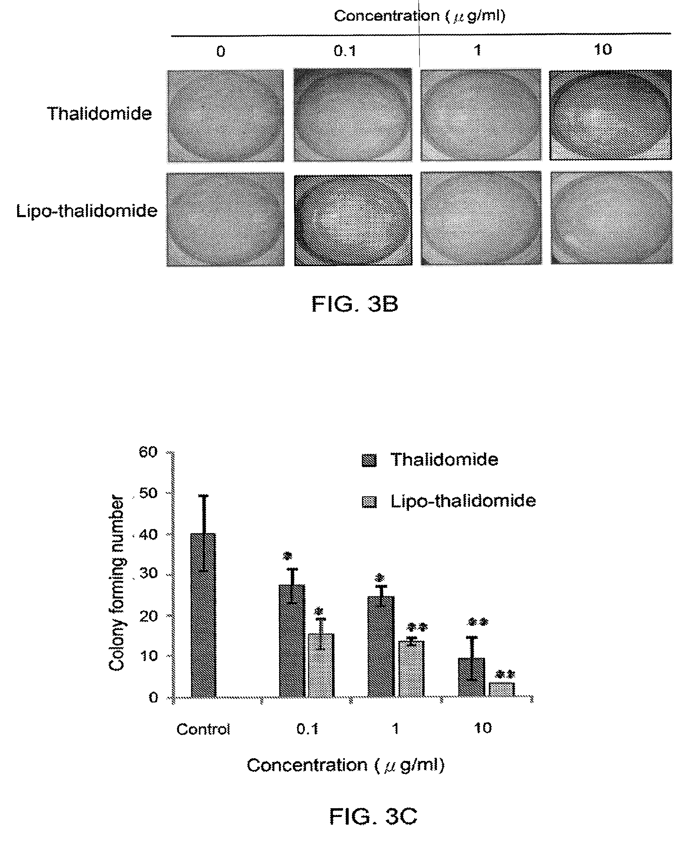

FIG. 3C shows inhibition of anchorage-independent growth of U-87 MG cell by thalidomide. Cells were cultured in soft agar containing free-form or liposomal Thalidomide (0∼10 µg/ml). Colonies were counted 14 days after the start of the relevant experiment.

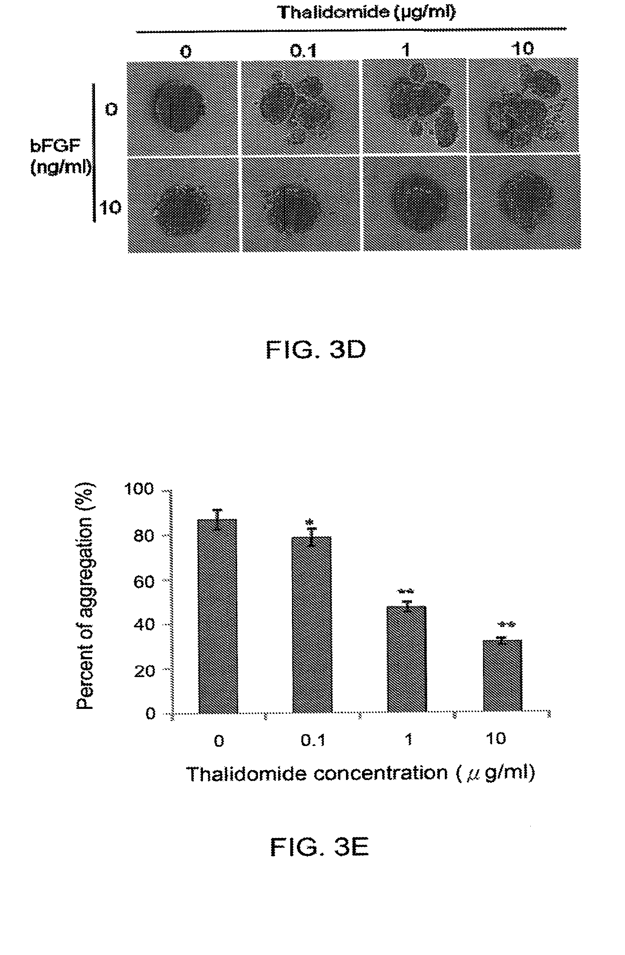

FIG. 3D shows disaggregation of spheroids by thalidomide, and reversal of thalidomide disaggregation effect by bFGF. Cells were suspended in culture medium containing 0∼10 µg/ml of thalidomide with or without exogenous bFGF. Spheroids were photographed by phase-contrast microscopy. The magnification was 100.
FIG. 3E shows inhibition of three-dimension growth of U-87 MG cells by thalidomide. Cells were suspended in culture medium containing 0∼10 µg/ml of thalidomide. The percentage of aggregation was analyzed.

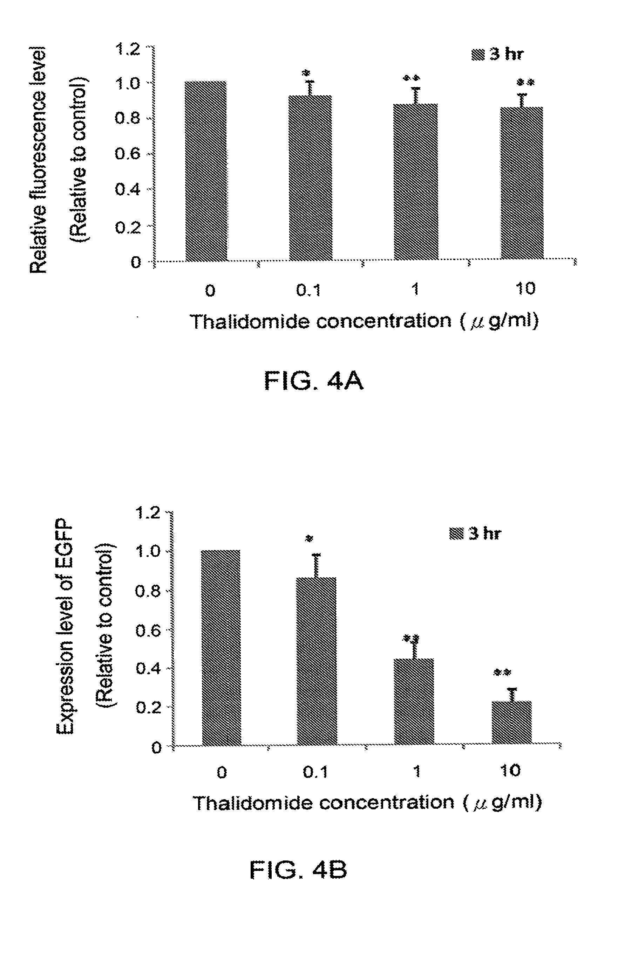

FIG. 4A shows inhibition of bFGF promoter-controlled EGFP reporter gene expression by thalidomide in U-87 MG cells. The cells were stably transfected with plasmid pbFGF-EGFP. After 0∼10 µg/ml thalidomide treatment for 3 hr, EGFP transcript expression levels were determined by flow cytometry.
FIG. 4B shows inhibition of bFGF promoter-controlled EGFP reporter gene expression by thalidomide in U-87 MG cells. The cells were stably transfected with plasmid pbFGF-EGFP. After 0∼10 µg/ml thalidomide treatment for 3 hr, EGFP transcript expression levels were determined by real-time PCR analysis.

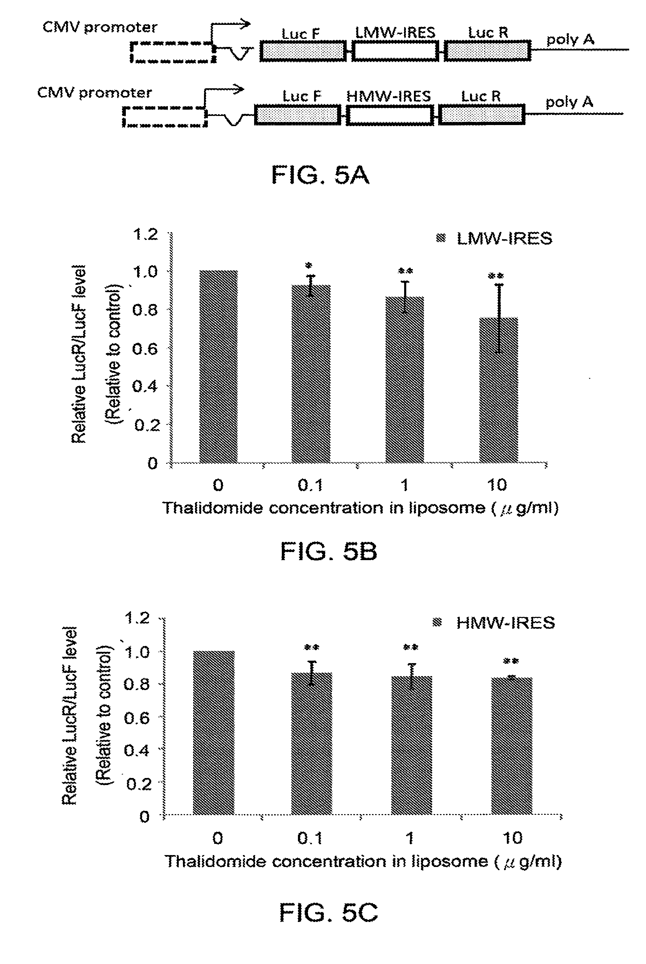

FIG. 5A is a schematic representation of the plasmid pLMW-IRES and pHMW-IRES.
FIG. 5B shows inhibition of LMW-IRES-dependent translation by thalidomide in U-87 MG cells. Cells were stably transfected with the bicistronic vector pLMW-IRES from FIG. 5A and treated with 0-10 µg/ml thalidomide for 12 hr. The IRES activity was determined by calculating the LucR/LucF ratio.
FIG. 5C shows inhibition of HMW-IRES-dependent translation by thalidomide in U-87 MG cells. Cells were stably transfected with the bicistronic vector pHMW-IRES from FIG. 5A and treated with 0∼10 µg/ml thalidomide for 12 hr. The IRES activity was determined by calculating the LucR/LucF ratio.

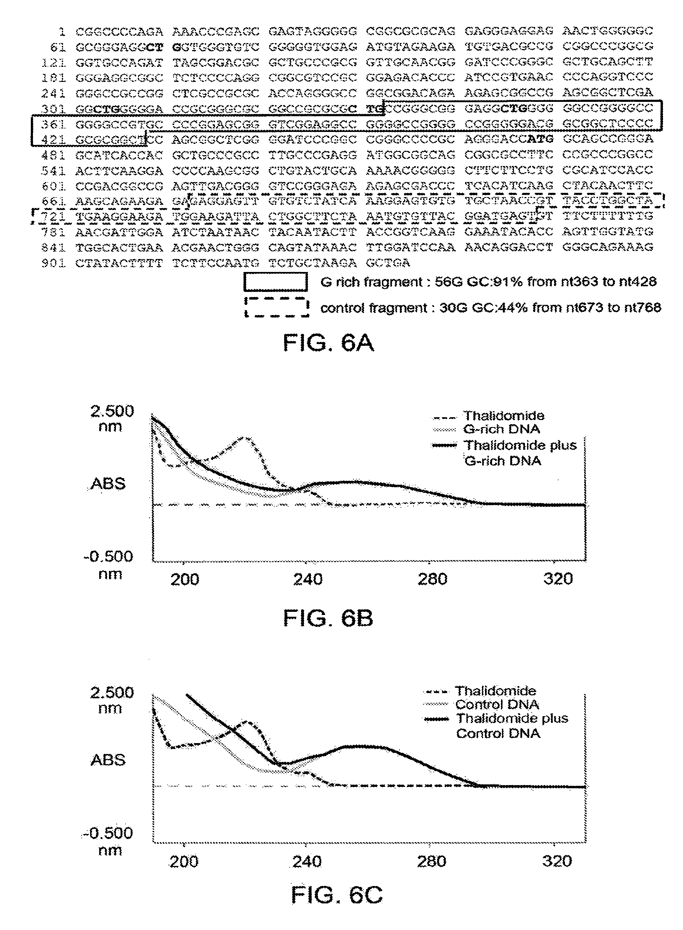

FIG. 6A shows partial bFGF cDNA sequence. The G-rich fragment is marked by a solid line box and non-G-rich control DNA fragment marked by a dotted line box.
FIG. 6B shows a UV-VIS absorbance spectrum of thalidomide after incubation with G-rich bFGF DNA fragment.
FIG. 6C shows a UV-VIS absorbance spectrum of thalidomide after incubation with non-G-rich bFGF control DNA fragment.

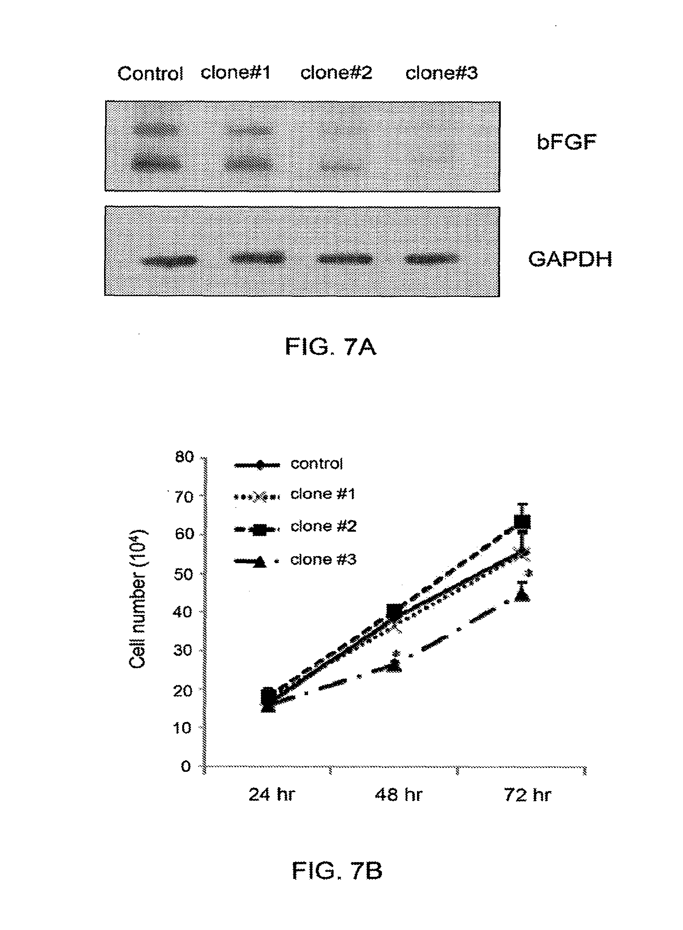

FIG. 7A is a western blot showing in bFGF knock-down clones and control clone, the expression levels of bFGF protein were dramatically reduced compared with those of the internal control GAPDH. Clone Nos. 1∼3 represent those clones which were derived from U-87 MG cells expressing bFGF shRNA Nos. 1∼3, respectively.
FIG. 7B shows cell proliferation ability of bFGF knock-down clones and control clone.

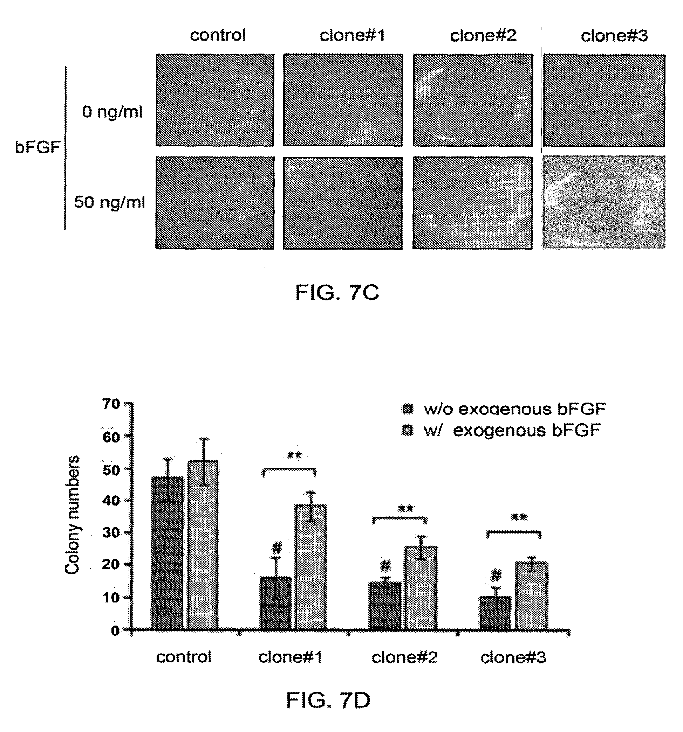

FIG. 7C shows inhibition of anchorage-independent growth of bFGF knock-down clones by thalidomide and recovery by exogenous bFGF treatment. Cells were cultured in soft agar containing free-form or liposomal thalidomide (0∼10 µg/ml) with or without exogenous bFGF. Colonies were photographed 14 days later.
FIG. 7D shows inhibition of anchorage-independent growth of bFGF knock-down clones by thalidomide and recovery by exogenous bFGF treatment. Cells were cultured in soft agar containing free-form or liposomal thalidomide (0∼10 µg/ml) with or without exogenous bFGF. Colonies were counted 14 days later.

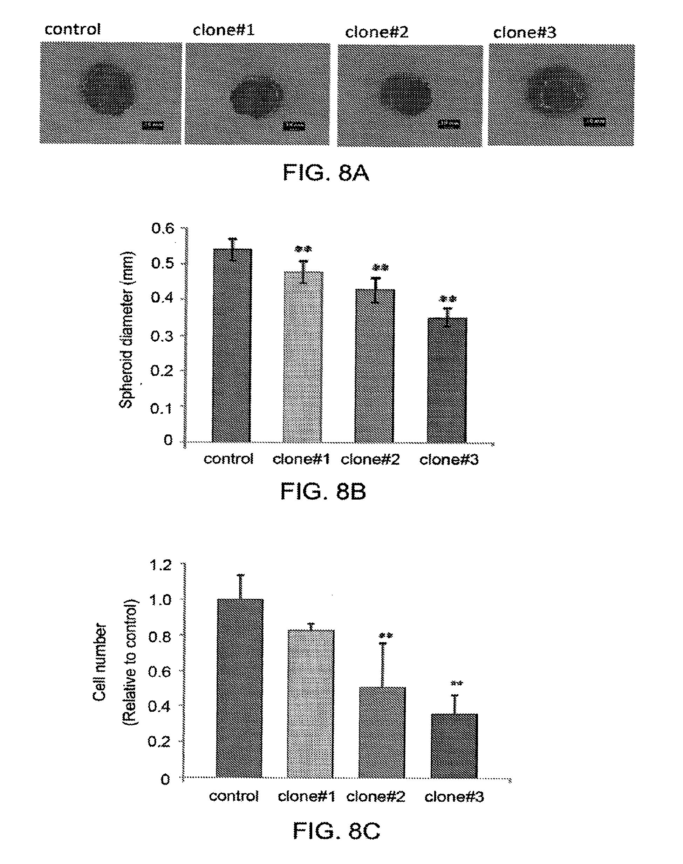

FIG. 8A shows morphology of spheroids from bFGF knock-down clones and control clone. Spheroids were photographed by phase-contrast microscopy.
FIG. 8B shows the diameters of spheroids from bFGF knock-down clones and control clone.
FIG. 8C shows the number of cells in the spheroids from bFGF knock-down clones and control clone.

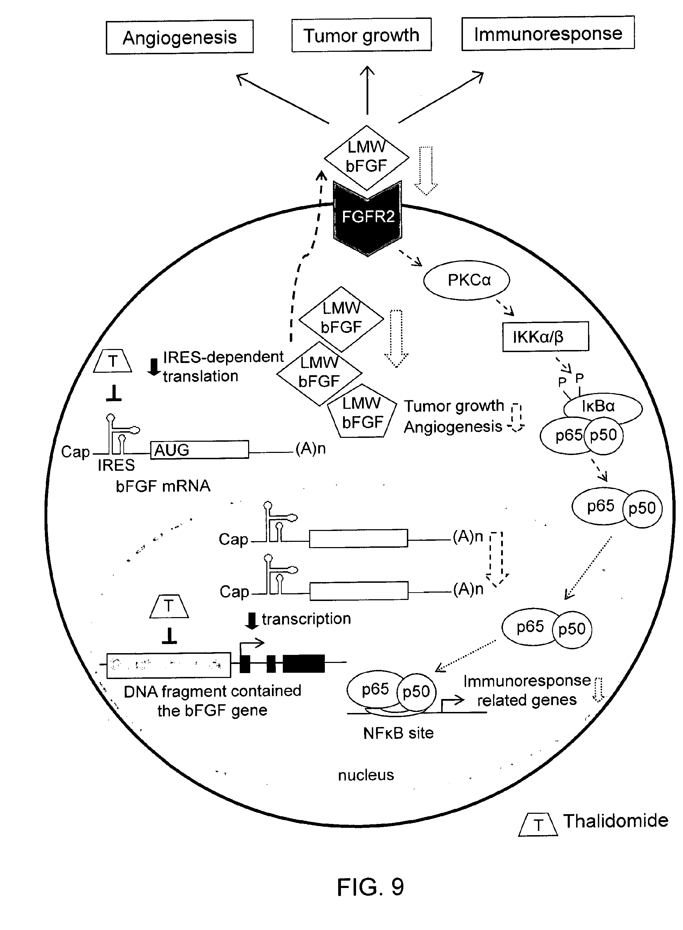

FIG. 9 is a schematic drawing showing bFGF expression would be regulated by thalidomide on at least two levels.

### DETAILED DESCRIPTION OF THE INVENTION

### DEFINITIONS

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

Unless otherwise defined, all technical and scientific terms used herein have the same meaning as commonly understood by one of ordinary skill in the art to which this invention pertains. In the case of conflict, the present document, including definitions will control.

As used herein, "around", "about" or "approximately" shall generally mean within 20 percent, preferably within 10 percent, and more preferably within 5 percent of a given value or range. Numerical quantities given herein are approximate, meaning that the term "around", "about" or "approximately" can be inferred if not expressly stated.

### OVERVIEW OF THE INVENTION

In a preferred embodiment of the present application, it is showed that thalidomide downregulated the expression of bFGF RNA transcripts by targeting its G- and/or GC-rich promoter in U-87 MG human glioma cells at the relatively low concentration of 0.1 µg/ml even lower than the prior clinical therapeutic serum concentrations of 1.8-10 µg/ml (Eleutherakis-Papaiakovou *et al*., 2004). A preferred embodiment also shows that thalidomide down-regulated the expression of different bFGF isorbrms in a dose-dependent manner (0.1, 1, 10 µg/ml), which is resulting from the change of the G-and/or GC-rich IRES activity. Because thalidomide had been reported to be highly susceptible to hydrolysis in solution (Eriksson *et al*., 1998), the present application further provides a method for increasing the bio-availability of thalidomide at the concentration between 0.1 to 10 µg/ml by a slow-release technology, such as encapsulated by liposome.

A preferred embodiment implicated the G- and/or GC-rich promoter and/or G- and/or GC-rich coding sequence of bFGF are the major targets of thalidomide. By applying thalidomide as a research tool, it is also possible to find out that bFGF may play a very important role in tumor anchorage-independent growth, which is a hallmark of tumorigenicity. The molecular mechanism of thalidomide provided in the preferred embodiment of the present application offers a new way for the arrest of cancers, angiogenesis-associated diseases, immunological disorders and sleep disorders in a relative lower therapeutic dose using drug delivery technologies, such as those performed by liposome, N-trimethyl chitosan and pH-dependent sustained release, and especially provides the useful indicator for treating diseases with high bFGF expression level instead of random clinical trials.

### EXAMPLES

Without intent to limit the scope of the invention, exemplary instruments, apparatus, methods and their related results according to the embodiments of the present invention are given below. Note that titles or subtitles may be used in the examples for convenience of a reader, which in no way should limit the scope of the invention. Moreover, certain theories are proposed and disclosed herein; however, in no way they, whether they are right or wrong, should limit the scope of the invention so long as the invention is practiced according to the invention without regard for any particular theory or scheme of action.

### EXAMPLE 1

### Thalidomide down-regulates bFGF RNA levels in U-87 MG cells

To examine whether the anti-tumor effect of thalidomide is via down-regulating the expression of bFGF, a high grade human glioma U-87 MG cell line was used due to its highly basal level of bFGF (Ke *et al*., 2000). The U-87 MG cells were purchased from American Type Culture Collection (ATCC, Rockville, MD) and maintained in Dulbecco's modified Eagle's medium (DMEM; Gibco) containing 10% heat inactivated fetal bovine serum (FBS; Gibco) and antibiotics, such as penicillin G (Sigma-Aldrich) and streptomycin (Sigma-Aldrich), at 37°C in a humidified incubator of 5% CO₂-95% air. Thalidomide (TYY Biopharm, Taiwan) was dissolved in dimethyl sulfoxide (DMSO; Sigma-Aldrich) first to make a stock solution of 50 mg/ml, and then diluted to various, desired concentrations with medium. The maximum of the final concentration of DMSO in the medium was 0.02%.

Real-time RT-PCR analysis was used to assess the RNA levels of bFGF in U-87 MG cells. After being treated with indicated concentrations (0, 0.1, 1 and 10 µg/ml) of thalidomide for 3 hr and 12 hr, U-87 MG cells were washed twice with ice-cold phosphate buffered saline (PBS) and RNA was extracted by using RNA-Bee™ RNA isolation solvent (Tel-test). Total RNA (5 µg) was used to prepare cDNA by using AMV reverse transcriptase (Promega). The reverse-transcribed cDNA samples were analyzed by real-time PCR using ABI Prism 7700 Sequence Detection System (Applied Biosystems) and the SYBR Green Master Mix kit (Applied Biosystems). Real-time PCR primers targeting human glyceraldehyde 3-phosphate dehydrogenase (GAPDH) primers (SEQ ID NO. 1 and SEQ ID NO. 2), bFGF primers (SEQ ID NO. 3 and SEQ ID NO. 4) were designed using Primer Express software (Applied Biosystems), and primers' sequences are shown in Table 1.

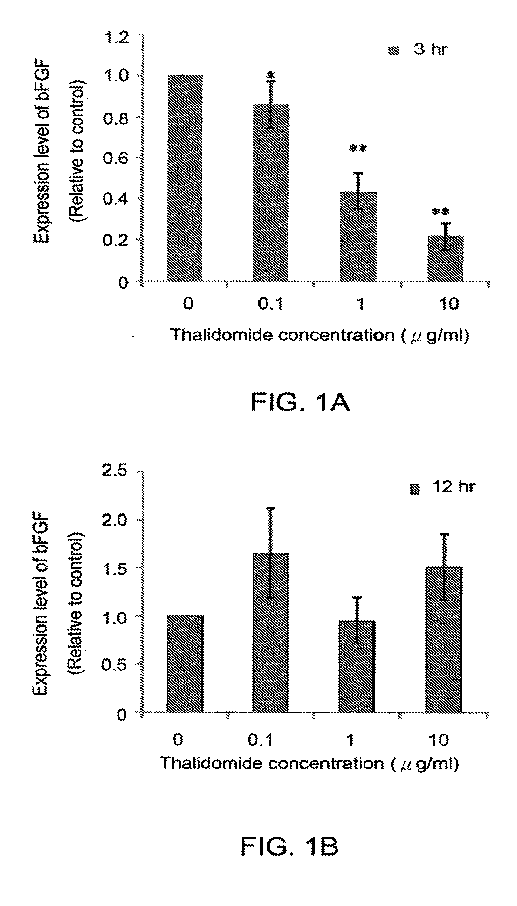

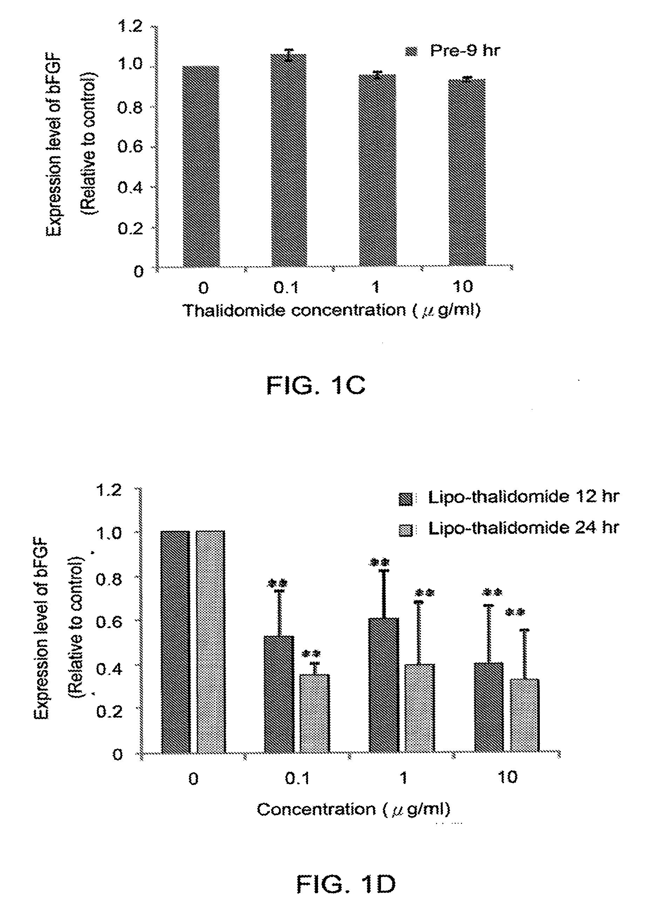

The PCR condition is as follows: 95°C denaturation for 10 min followed by 40 cycles of 95°C for 15 sec, 55°C for 20 sec, and 72°C for 40 sec. The expression level of human GAPDH was used as an internal reference. Relative gene expression levels were calculated with the 2^{-ΔΔCT}, bFGF RNA levels in U-87 MG cells were markedly reduced after being treated with 0.1∼10 µg/ml thalidomide for 3 hr (FIG. 1A) even at concentrations lower than the reported therapeutic dose (3-6 µg/ml) (Vacca *et al.,* 2005). However, when cells were treated with thalidomide for longer periods (12 hr), its inhibitory effect on bFGF expression disappeared (FIG. 1B).

**Table 1**

| Name | Sequence | SEQ ID NO. |
|---|---|---|
| GAPDH-F | 5'-AATGTCACCGTTGTCCAGTTG-3' | 1 |
| GAPDH-R | 5'-GTGGCTGGGGCTCTACTTC-3' | 2 |
| bFGF-F | 5'- ATCAAAGGAGTGTGTGCTAACC-3' | 3 |
| bFGF-R | 5'-ACTGCCCAGTTCGTTTCAGTG-3' | 4 |
| bFGF promoter-F | 5'-GTGGCACCTGCTATATCCTACTG-3' | 5 |
| bFGF promoter-R | 5'-AGCCTCGAGCCGCTCGG-3' | 6 |
| EGFP-F | 5'-CCATGGTGAGCAAGGGCGAG-3' | 8 |
| EGFP-R | 5'-TCAGGGTCAGCTTGCCGTAGG-3' | 9 |
| LMW-IRES-F | 5'-CTCCTGACGCGGGGCCGTGCCCCGGAGCGG-3' | 10 |
| LMW-IRES-R | 5'-CTCACAACGGGTTGTGAGGGTCGCTCTTCT C-3' | 11 |
| HMW-IRES-F | 5'-CTCCTGACGCGTCAGGAGGGAGGAGGACTG G-3' | 13 |
| HMW-IRES-R | 5'-CTCACAACGGGTTGTGAGGGTCGCTCTTCT C-3' | 14 |

In order to test the stability of thalidomide in the culture medium alone, the thalidomide stock solution was diluted with fresh culture medium to 0.1∼10 µg/ml and incubated at 37°C in a humidified incubator of 5% CO₂-95% air for 9 hr before being added to the U-87 MG cells for 3 hr. As shown in FIG. 1C, thalidomide completely lost its activity even after a short (9 hr) incubation in culture media. In order to increase the stability of thalidomide in aqueous solution, thalidomide could be encapsulated by a vehicle or pharmaceutical acceptable carriers, such as liposome and N-trimethyl chitosan. Thalidomide was encapsulated by liposome to form liposomal thalidomide according to the method described previously (Fang *et al*., 2005) with modifications. Briefly, egg phosphatidylcholine (120 mg; Fluka) and cholesterol (30 mg; Sigma) in the ratio of 4 to 1 by weight and 12 mg thalidomide were mixed together, dissolved in 5 ml of a chloroform-methanol solution (2:1, v/v), and then evaporated in a rotary evaporator at 40°C. Solvent traces were removed by maintaining lipid films under a vacuum for overnight. The films were first hydrated with 10 ml distilled water in a bath-type sonicator at 4°C for 1 hr. The aqueous dispersion of liposome was further homogenized with a probe-type sonicator to give a smaller size of liposome, followed by filteration through a series of nylon meshes of 74, 53, 30 and 10 µm pore size, and then centrifuged at 26,000×g to collect the liposome pellet. The liposomal thalidomide was dissolved in methanol and its UV absorbance measured at 230 nm so as to determine the concentration of the liposomal thalidomide. Interestingly, significant inhibition of bFGF transcripts in U-87 MG cells by liposomal thalidomide was detectable even after treating for 24 hr (FIG. ID), but the dose response observed earlier (FIG. 1 A) was no longer seen.

### EXAMPLE 2

### Thalidomide is sustained release via N- trimenthyl chitosan encapsulation

N-trimethyl chitosan (TMC) was synthesized as previously described (Thanou *et al*., 2000). Briefly, chitosan (2g; Sigma-Aldrich) was sieved through nylon meshes of 300 µm pore size and mixed with sodium iodide (4.8g; Sigma-Aldrich) in 15% (w/v) sodium hydroxide (11 ml; NaOH, Sigma-Aldrich), iodomethane (11.5 ml; Sigma-Aldrich) and 1-methyl-2-pyrrolidinone (80 ml; Sigma-Aldrich) at 60°C for 75 min. The product was precipitated by 4 volume 95% (v/v) ethanol, isolated by centrifugation at 1670 ×g and thoroughly washed with ether to remove ethanol. Then the obtained product was dissolved in 1-methyl-2-pyrrolidinone (80 ml; Sigma-Aldrich) at 60°C to remove ether and then mixed with sodium iodide (4.8g; Sigma-Aldrich) in 15% (w/v) (11 ml; NaOH, Sigma-Aldrich) and iodomethane (11.5 ml; Sigma-Aldrich) at 60°C for the secondary step of reductive methylation. The product was precipitated by addition of 4 volume 95% (v/v) ethanol, isolated by centrifugation at 1670 ×g and thoroughly washed with ether. The purification steps included that ach product was dissolved in 10% (w/v) sodium chloride (20 ml; NaCl, J.T. Baker) to exchange the iodide with chloride, precipitated by 4 volume 95% (v/v) ethanol, isolated by centrifugation at 1670 ×g, thoroughly washed with ether and dialyzed against deionized water overnight. The TMC was dried in vacuo and measured its characterization in D₂O by a 500-MHz spectrometer (Bruker Avance 500 MHz NMR). The nanoparticles of thalidomide encapsulated by TMC were prepared using a ionic-gelation method under magnetic stirring at room temperature as previously described (Mi *et al*., 2008). In brief, thalidomide (18.16 mg/ 31.91 mL (deionized H₂O/ethanol=2/3, v/v)) was premixed with an aqueous poly(γ-glutamic acid) (18.26 mg/1.97 ml deionized H₂O; Vedan, Taiwan). Subsequently, magnesium sulfate (36.54 mg/4.12 mL deionized H₂O; MgSO₄, Sigma-Aldrich) was blended into the mixture and thoroughly stirred for 1 hr. An aqueous TMC (114.6 mg/20mL deionized H₂O) was added into the mixed solution under magnetic stirring at room temperature for 1 hr. In order to determine the loading content and loading efficiency, nanoparticles of thalidomide encapsulated by TMC were collected by centrifugation at 45,000 rpm (227480 in a Beckman 55.2 Ti rotor (Beckman Coulter) and assayed by liquid chromatography Mass (LC/MS/MS; Bruker). Compared with rapid hydrolysis of thalidomide as previous report (Eriksson *et al*., 1998), thalidomide encapsulated by TMC could be released sustainedly.

### EXAMPLE 3

### Thalidomide down-regulates bFGF protein levels and its nuclear distribution

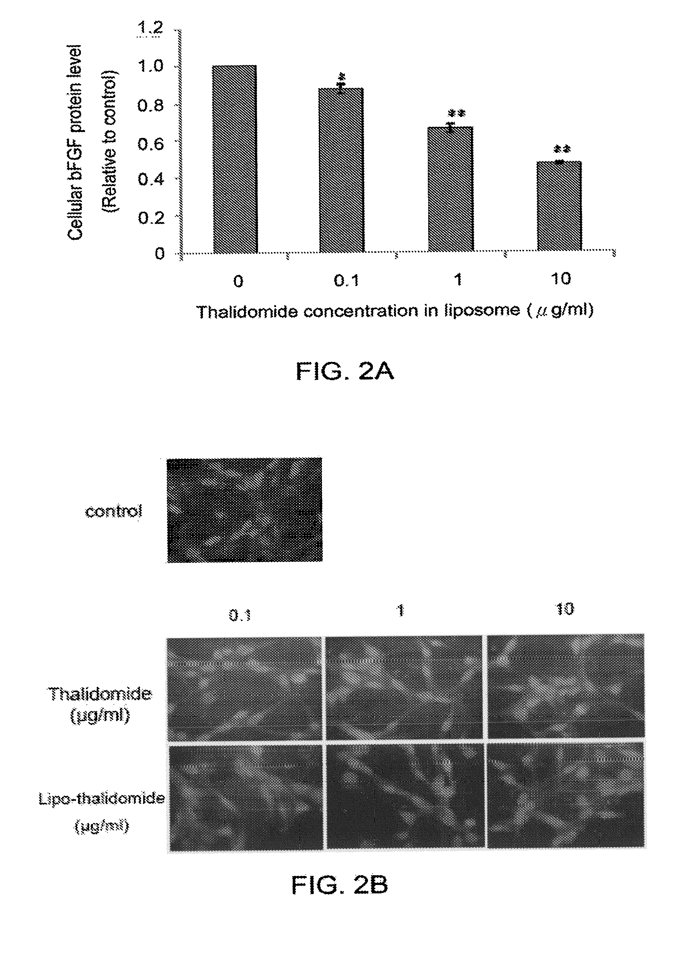

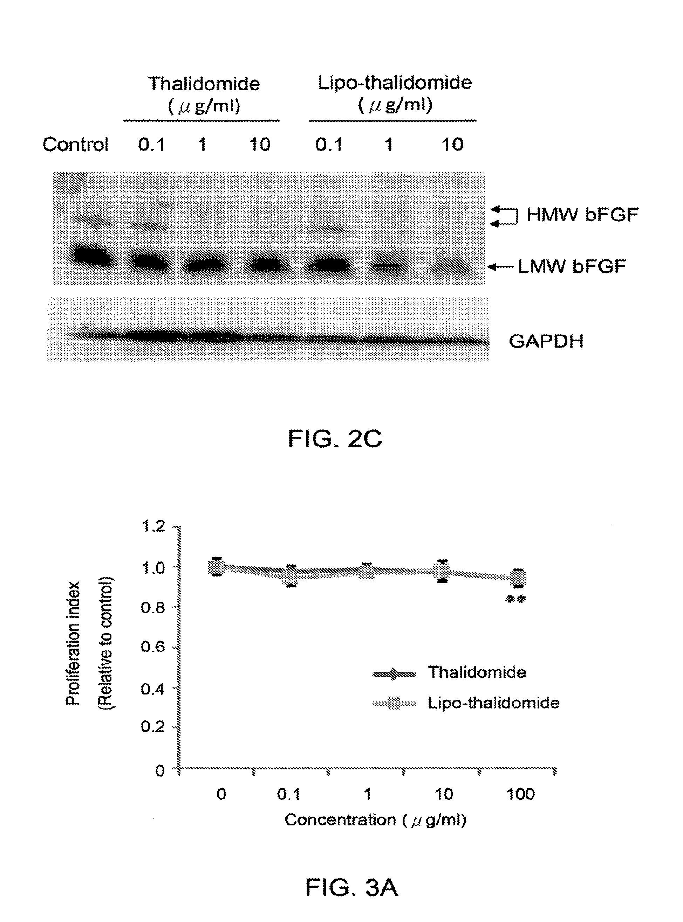

U-87 MG cells were treated with indicated concentrations (0, 0.1, 1 and 10 µg/ml) of free-form and liposomal thalidomide and fixed in 4% (w/v) paraformaldehyde (Sigma-Aldrich) in PBS for 15 min, permeabilized with 0.01% (v/v) Triton X-100 (Sigma-Aldrich) or 0.5 % (v/v) saponin (Sigma-Aldrich) in PBS for 30 min at room temperature. The cells were subsequently treated with 0.5 µg polyclonal rabbit anti human bFGF antibody (ab16828, Abeam) for 30 min at room temperature, washed, followed by staining with FITC-conjugated goat anti-rabbit IgG (Jackson ImmunoResearch) at 1:200 dilution for 30 min. Hoechst 33258 (Sigma-Aldrich) was used as a nuclear marker. The cells were then washed and visualized using a fluorescence microscope (Olympus Optical Co, Tokyo, Japan) or analyzed using the BD FACSCalibur™ flow cytometer (BD Biosciences). The relative expression level of cellular bFGFs was calculated by normalized the mean fluorescence value of each treatment with the empty liposome treated control. The Immunofluorescence stainings showed that thalidomide down-regulated not only total (FIG. 2A) but also nuclear (FIG. 2B) level of bFGF proteins. Because HMW bFGFs were the major isoforms localized in nucleus (Renko *et al*., 1990), a decrease of its signal intensity in this compartment might reflect a reduced expression of HMW bFGFs.

Immunoblot (or western blot) analysis was therefore performed to analyze the amount of different bFGF isoforms and GAPDH was used as the internal control, which level would not be affected by thalidomide. After being treated with indicated concentrations (0, 0.1, 1 and 10 µg/ml) of free-form and liposomal thalidomide, U-87 MG cell lysate was prepared using lysis buffer (50 mM Tris [hydroxymethyl] aminomethane (Tris; USB), 1% (v/v) TritonX-100 (Sigma-Aldrich), 150 mM sodium chloride (NaCl; J.T.Baker), 1mM ethylenediaminetetraacetic acid (EDTA; Sigma-Aldrich), 1mM phenylmethylsulphonyl fluoride (PMSF; Sigma-Aldrich). Cell lysates (2µg) containing proteins were separated with using 15% polyacrylamide gel electrophoresis and transferred to a polyvinylidene fluoride (PVDF) membrane (PerkinElmer). The membrane was incubated with polyclonal rabbit anti human bFGF antibody (ab 16828, Abeam) at 1:200 dilution or anti-GAPDH antibody (ab9482, Abeam) at 1:10000 dilution, which was used as an internal control, followed by horseradish peroxidase-conjugated anti-IgG secondary antibody (Jackson ImmunoResearch) at 1:5000 dilution. The enhanced chemiluminescent (ECL; PerkinElmer) detection method (Amersham) was used for blotting analysis. Without treatment of thalidomide, U-87 MG expressed all bFGF isoforms, but the 24-kilodalton (kDa) one was lower than the other forms (FIG. 3C). Even though both HMW and LMW bFGFs were translated from the same transcript, a decrease of the HMW bFGFs induced by thalidomide was more dramatic than that of LMW ones (1 and 10 µg/ml), while liposomal thalidomide could down-regulate not only the level of HMW bFGF but also the level of LMW bFGF dose-dependently (FIG. 3C).

### EXAMPLE 4

### Effects of thalidomide on cell proliferation and anchorage-independent growth

Since thalidomide could down-regulate bFGF expression in U-87 MG cells, we next evaluated its effects on their growth because overexpression of this growth factor in glioma cells was reported to stimulate their proliferation in an autocrine manner, and the introduction of bFGF antisense oligonucleotides in these cells could block their growth and colony formation in soft agar (Murphy *et al.*, 1992). Cell proliferation ability was assayed using a resazurin assay (Nociari *et al*., 1998), in which resazurin dye was used as a redox indicator to detect cell growth, not cell death. Resazurin sodium (Sigma-Aldrich) stock solution in PBS (5 mM) was prepared, and the working solution (50µM) was diluted from the stock using DMEM (Gibco) without FBS. Approximately 3000 U-87 MG cells were seeded onto 96-well plates (Costar, Corning), allowed to attach at 37°C in a humidified incubator of 5% CO₂-95% air for 16 hr, and treated with indicated concentrations (0, 0.1, 1, 10, and 100 µg/ml) of free-form and liposomal thalidomide for 72 h. For resazurin assay, the culture medium was removed and freshly diluted resazurin working solution (100 µl) was added into each well. Following incubation at 37°C in a humidified incubator of 5% CO₂-95% air for 2 hr, the resazurin dye was reduced by the activity of living cells, and the reduced form of resazurin was determined at a fluorescence excitation wavelength 530 nm and emission wavelength 590 nm by a Victor 2 1420 Multilable Counter (Wallac, PerkinElmer). As shown in FIG. 3A, only high concentration (100 µg/ml) of liposomal thalidomide could slightly reduce the proliferation of U-87 MG cells.

Because bFGF was known to promote cell transformation (Vagnuer *et al*., 1996), the soft agar colony formation assay (Murphy *et al.,* 1992) and hanging drop method (Kelm *et al*., 2003) were used to assess the effects of thalidomide on anchorage-independent and three-dimensional growth abilities of U-87 MG cells, respectively. The colony forming assay was performed according to a two-layer agar technique (Murphy *et al*., 1992). The bottom layer consisted of 0.3 ml of DMEM with 10% FBS and 0.5% (w/v) agarose (Amresco). Approximately 1000 U-87 MG cells were added to the same medium containing 10% FBS and 0.3% (w/v) low-melting agarose (Amresco) plus indicated concentrations (0, 0.1, 1 and 10 µg/ml) of free-form thalidomide, and plated in 24-well plates (Costar, Coming) onto the base layer. After 2 weeks of incubation at 37°C in a humidified incubator of 5% CO₂-95% air, cells were stained with 3-(4,5-dimethylthiazol-2-yl)-2,5-diphenyltetrazolium bromide (MTT; Sigma-Aldrich) dye solution and plates were photographed and colonies numbers counted. Colony-forming ability (size, >0.1 mm) was measured. As shown in FIG. 3B and FIG. 3C, free-form and the lyposomal thalidomide were effective at low concentration (0.1 µg/ml) and clearly exhibited a dose-dependent response.

For hanging drop assay, approximately 1000 U-87 MG cells per 20 µl of cell suspension in culture medium (DMEM with 10% FBS) with indicated concentrations (0, 0.1, 1 and 10 µg/ml) of free-form thalidomide were spotted on the cover of a 6-cm culture dish (Falcon). The cover was returned to its top position with the cell suspension droplet facing down toward the bottom dish, which contained 5 ml of DMEM for maintenance of moisture during incubation. The spheroids were formed for 48 hr after the incubation at 37°C in a humidified incubator of 5% CO₂-95%) air. Each spheroid was photographed by using phase-contrast microscopy. The aggregation percentage was assayed by calculating the aggregation ability from 20 spheroids for each assay condition. The cell aggregates formed in spheroid culture was abolished by free-form thalidomide dose-dependently (FIG. 3D and FIG. 3E).

### EXAMPLE 5

### Thalidomide down regulates bFGF transcription by regulating its G- and/or GC-rich promoter

To evaluate the effect of thalidomide on the transcription driven by bFGF promoter, pbFGF-EGFP plasmid, containing bFGF promoter to drive the expression of enhanced green fluorescence protein (EGFP), was stably transfected into U-87 MG to get U87-bFGF-EGFP cell. Briefly, genomic DNA was purified from U-87 MG cells. About 500 ng genomic DNA was used as template and amplification of PCR fragments were performed on ABI 2700 thermocycler by using Taq polymerase (GENET BIO). The primers (SEQ ID NO. 5 and SEQ ID NO. 6) used for amplifying bFGF promoter was showed in Table 1. The PCR condition was 96°C for 10 min followed by 35 cycles of 95°C for 40 sec, 58°C for 40 sec, and 72 °C for 1 min, and thereafter 72°C for 7 min and then kept at 4 °C. The bFGF promoter fragments (SEQ ID NO. 7) were cloned into pGEMT-easy vector (Promega) and subcloned into pEGFP-N2 vector (BD Biosciences Clontech) to generate plasmid pbFGF-EGFP. Approximately 1 × 10⁵ U-87 MG cells were plated in 6-well plates (Falcon) 24 hr before transfection and exposed to 3 µg total DNA (plasmid pbFGF-EGFP) and 3 µl Lipofectamine 2000 (Invitrogen Corp.) in DMEM without FBS according to the manufacture's brochure of Lipofectamine 2000. After cultured at 37°C in a humidified incubator of 5% CO₂-95% air for 48 hr, cells were trypsinized and passaged into DMEM with 10% FBS (20X dilutions). Stable transfectants (U87-bFGF-EGFP cells) were selected by using geneticin (800 µg/ml; Merck Biosdences) for 1 month.

U87-bFGF-EGFP cells were treated with indicated concentrations (0, 0.1, 1 and 10 µg/ml) of free-form thalidomide for 3 hr. The fluorescence of EGFP was measured by flow cytometry (FACS Calibur, BD Biosciences). The relative fluorescence indexes were measured to evaluate the effect of thalidomide on the expression of EGFP controlled by bFGF promoter. The RNA levels of EGFP were analyzed by real-time RT-PCR and GAPDH was used as an internal control. The EGFP primers (SEQ ID NO. 8 and SEQ ID NO.9) are shown in Table 1. Thalidomide was shown to diminish EGFP transcripts and the fluorescence in a dose-dependent pattern after 3 hr treatment (FIG. 4A and FIG. 4B).

### EXAMPLE 6

### Thalidomide down regulates bFGF translation by modulating its IRES activity

To examine whether the G- and/or GC-rich region in IRES of N-terminal extension of bFGF transcript could also be regulated by thalidomide, plasmids pHMW-IRES and pLMW-IRES (FIG. 5A) were designed using bicistronic vector as previous described (Creancier *et al*., 2000). There were two luciferase genes, *Renilla* luciferase (LucR) and firefly luciferase (LucF), which were controlled by the cytomegalovirus (CMV) promoter and separated by the LMW-IRES fragment (SEQ ID NO. 12) and HMW-IRES fragment (SEQ ID NO. 15) in plasmids pLMW-IRES or pHMW-IRES (FIG. 5A), respectively. The LMW-IRES fragment (SEQ ID NO. 12) and HMW-IRES fragment (SEQ ID NO. 15) were amplified from U-87 MG genomic DNA by PCR with the primers (SEQ ID NO. 10, SEQ ID NO. 11, SEQ ID NO. 13 and SEQ ID NO. 14) showed in Table 1, then cloned into pGEMT-easy vector (Promega) and subcloned into a bicistronic vector system (Promega) to generate plasmids pLMW-IRES and pHMW-IRES, respectively. Approximately 2 × 10⁵ U-87 MG cells were plated in 6-well plates (Falcon) 24 hr before transfection and exposed to 3 µg total DNA (plasmid pLMW-IRES or pHMW-IRES) and 3 µl Lipofectamine 2000 (Invitrogen, Carlsbad, USA) in DMEM without FBS according to the manufacture's brochure of Lipofectamine 2000. After cultured at 37°C in a humidified incubator of 5% CO₂-95% air for 48 hr, cells were trypsinized and passaged into DMEM with 10% FBS (20X dilutions). Stable transfectants (U87-HMW-IRES and U87-LMW-IRES cells) were selected by using geneticin (800 µg/ml; Merck Biosciences) for 1 month.

U87-LMW-IRES and U87-HMW-IRES cells were treated with indicated concentrations (0, 0.1, 1 and 10 µg/ml) of liposomal thalidomide for 12 hr, and the two luciferase activities were measured in each cell extracts by scintillation counting in a Victor 2 1420 Multilabel Counter (Wallac, PerkinElmer). The IRES activity was determined by calculating the LucR/LucF ratios (the ratio of *renilla* to firefly luciferase acitivity) normalized by the untreated control. Thalidomide did alter the IRES activity in a dose-dependent manner for both LMW-IRES and HMW-IRES (FIG. 5B and FIG. 5C).

### EXAMPLE 7

### The UV-VIS absorbance of thalidomide is more effectively quenched by a G-rich DNA fragamen

The GC content of large genomic DNA (>100 kb) ranges from 30% to 65% and the average is about 40% (Venter *et al.,* 2001; Lander *et al..* 2001). Nucleic acid with GC content more than 50% would be G- and/or GC-rich. The G-rich fragment with 91% GC content and non-G-rich control fragment with 44% GC content were designed from the promoter region of bFGF (FIG. 6A). To examine whether thalidomide could interact preferentially with the G- and/or GC-rich region of bFGF, the ultraviolet-visible (UV-VIS) absorbance of thalidomide was assayed using a Hitachi U2000 Spectrophotometer with the scanning range from 330 to 190 nm. The absorbance of thalidomide would be diminished by bound tightly with the secondary structure of a DNA (Usha *et al*., 2005). As shown in FIG. 6B and FIG. 6C, a more severe quench of the absorbance at 230 nm of thalidomide was detected when it was incubated with a G-rich fragment than with non-G-rich control fragment, and this result suggested that thalidomide might bind preferentially with nucleic acids that have a high content of GC.

### EXAMPLE 8

### Anchorage-independent growth of U-87 MG cells is suppressed by knocking down its bFGF expression

It has been demonstrated that thalidomide not only down-regulated bFGF expression in U-87 MG cells but also inhibited their colony formation in soft agar, then the tumorigenicity of these cells was examined to determine whether it was reduced by knocking down its bFGF expression.

Recombinant lentiviruses were produced by transient transfection of human embryonic kidney cell line 293T cells (ATCC, Rockville, MD) using the ecalcium-phosphate method according to the guideline provide by the National RNAi Core Facility (Institute of Molecular Biology / Genomic Research Center, Academia Sinica, supported by the National Research Program for Genomic Medicine Grants of NSC, Taiwan). Briefly, 293 T cells were cotransfected with 20 µg pLKO.1-puro lentiviral vector (National RNA Core Facility, Academia Sinica, Taipei, Taiwan) expressing non-target control shRNA (shGFP control, as shown in Table 2) (SEQ ID NO. 19) or bFGF shRNA (bFGF shRNA No. 1, No. 2, or No. 3, Table 2) (SEQ ID NO. 16, SEQ ID NO. 17 and SEQ ID NO. 18) along with 6 µg envelope plasmid pMDG (National RNAi Core Facility, Academia Sinica, Taipei. Taiwan) and 15 µg packaging plasmid pCMVΔR8.91 (National RNAi Core Facility, Academia Sinica, Taipei, Taiwan). Fresh culture medium (DMEM with 10% FBS) was replaced after 6 hr of the transfection. Infectious lentivruses were harvested at 40 and 64 hr post-transfection and filtered through 0.45 µm low protein binding filter (Millipore). The viral particles were spun down by ultracentrifugation (Beckman SW28 swingle bucket, 4°C, 2 h at 26,000 rpm). After centrifugation, the supernatants were discarded, and the viral pellets were suspended in 200 µl of FBS-free DMEM and stored at -70□.

To prepare bFGF knock-down cells, approximately 10⁶ U-87 MG cells in 5 ml DMEM with 10% FBS were plated into 6-cm culture dish (Falcon) and incubated at 37°C in a humidified incubator of 5% CO₂-95% air for 16 hr to allow cell attachment. The cells were then infected with lentivirus suspension (100 µl) for 24 hr. Because the reconbinant lentivirus had puromycin resistant gene, fresh medium (DMEM with 10% FBS) containing 1 µg/ml puromycin (Sigma-Aldrich) for knock-down cells selection was replaced every 3 days for 2 weeks. After selection, three bFGF knock-down clones from the respective shRNAs were selected and named as clone#1, clone#2 and clone#3.

**Table 2**

| Name | Sequence | SEQ ID NOs. |
|---|---|---|
| bFGF shRNA #1 | GCA GTC ATA AAC AGA AGA ATA | 16 |
| bFGF shRNA #2 | GAC CCT CAC ATC AAG CTA CAA | 17 |
| bFGF shRNA #3 | CTA TCA AAG GAG TGT GTG CTA | 18 |
| shGFP control | ACG TCT ATA TCA ATG GCC GAC A | 19 |

The western blot result shown that these three knock-down clones had different efficacies in down-regulating the expression of endogenous bFGF (FIG. 7 A). The cell proliferation activity of each clones showed no significant difference from that of the control clone except clone #3 (FIG. 7B). The anchorage-independent growth of these bFGF down-regulating cells was significant inhibited, especially the clone#3 (FIG. 7C). To distinguish between the contribution of LMW and HMW bFGF to the anchorage-independent growth of U-87 MG cells, aforementioned clones were incubated with recombinant LMW bFGF (0, 10, 50 and 250 ng/ml) before colonies formed in soft agar, which were then counted. Although the number of colonies formed from the bFGF down-regulating cells was increased significantly by LMW bFGF supplementation, the anchorage-independent growth abilities were only partially restored by this treatment (FIG. 7D and Table 3). The relevant results showed that nuclear bFGF (HMW ones) also plays an important role in cell transformation.

**Table 3**

| | recombinant human bFGF (ng/ml) | | | |
|---|---|---|---|---|
| | 0 | 10 | 50 | 250 |
| control | 46.8 ±6.3 | 52.0 ±7.0 | 57.8 ±5.2** | 58.8 ±7.0* |
| clone#1 | 16.0 ±6.7*** | 38.5 ±4.4* | 23.5 ±3.2*** | 21.0 ±7.3*** |
| clone#2 | 14.8 ±1.6*** | 25.8 ±3.4*** | 233 ±2.8*** | 27.2 ±7.6*** |
| clone#3 | 10.2 ±3.2*** | 20.8 ±2.1*** | 21.0 ±3.6*** | 21.7 ±4.4*** |

Results were expressed as the mean ± S.E. (n=6 per group). * P<0.005, ** p<0.01, *** p<0.001 vs. 0 ng/ml bFGF control cells (Student's *t* test).

### EXAMPLE 9

### The three-dimensional growth of U-87 MG was diminished after knock down cellular bFGF level

By using the hanging drop method to force tumor cells growing into spheroid, the time bFGF knock-down clones needed to aggregate was longer than control (date not shown), and the size of spheroids was shown to be significantly smaller (FIG. 8A and FIG. 8B). For further analysis, the spheroids were transferred into 96-well plates (Costar, Corning) containing 100 µl DMEM without FBS in each well. After allowed to attach at 37°C in a humidified incubator of 5% CO₂-95% air for about 12 hr and removal of the culture medium, the spheroids were stained with methylene blue (200 µl of 5 g/l in methanol; Sigma-Aldrich) for 30 min. The wells were washed for 5 times with tap water to remove the excess of dye and then the plates were allowed to dry overnight at 25°C. The stained spheroids were solved with 2% (w/v) SDS (200 µl/well; J.T.Baker) at 25°C for 24 hr. The viable cells in spheroid were expressed as a percentage of the methylene blue absorbance (at 650 nm) of spheroid lysates measured by PowerWave™ HT 340 (BioTek). The results showed that the cell number is also correlated with the cellular level of bFGF (FIG. 8C) and exogenous bFGF can accelerate cell proliferation ability and recovered the spheroid size in a dose-dependent manner (Table 4). It indicated that the ability of U-87 MG cells to grow into a three-dimensional spheroid is likely dependent on the endocrine machinery of bFGF.

**Table 4**

| | recombinant human bFGF (ng/ml) | | | |
|---|---|---|---|---|
| | 0 | 10 | 50 | 250 |
| Control | 1.00 ±0.14 | 1.02 ±0.10 | 1.08 ±0.20 | 1.49 ±0.20** |
| clone#1 | 0.83±0.04* | 0.99 ±0.14 | 0.99 ±0.18 | 1.08 ±0.30 |
| clone#2 | 0.51±0.25** | 0.69±0.24* | 0.89 ±0.4 | 1.10±0.18 |
| clone#3 | 0.36±0.11** | 0.42 ±0.1.4** | 0.73 ±0.21* | 0.85 ±0.15 |

| | | | | |
|---|---|---|---|---|
| Results were expressed as relative index of untreated control ± S.E. (n=8 per group). * p<0.05, ** p<0.01 vs. 0 ng/ml bFGF control cells (Student's *t* test). | | | | |

Thalidomide has been used and studied for more than 50 years, but its mechanisms of action are not fully understood. Many clinical trials of thalidomide were conducted based on its anti-angiogenic and immunomodulatory activities (Eleutherakis-Papaiakovou *et al.,* 2004). Interestingly, positive responses of some cancer patients to thalidomide have been shown to correlate with the changes in serum concentration of angiogenic factors such as VEGF, bFGF and HGF (Fine *et al.,* 2000; Neben *et al.,* 2001; Vacca *et al.,* 2005; Kakimoto *et al.*, 2002). In the present application, it is found that low concentration thalidomide was sufficient to down-regulate bFGF in U-87 MG cell dose-dependently and the bio-availability of thalidomide could be increased by a slow-release technology, such as being encapsulated with liposome, N-trimethyl chitosan and pH-dependent sustained release. In addition, the expression (FIG. 4A and FIG. 4B) and DNA binding analyses (FIG. 6B) of the present application suggested that the down-regulation of bFGF transcript levels by thalidomide is mediated by its direct interaction with the G-rich promoter of this gene. The effective concentrations of were much lower (0.1 and 1 µg/ml) than those (12.5 and 25 µg/ml) used by others to suppress the G-rich hTERT promoter (Drucker *et al.,* 2003). In the meantime, a decrease in bFGF protein levels was also found in these cells after thalidomide treatment which was associated with a change of nuclear localization of high molecular weight (HMW) bFGFs (FIG. 2B). The IRES activities present in both HMW and LMW bFGF transcripts (Bonnal *et al.,* 2003) were shown to be down regulated by thalidomide in a dose-dependent manner (FIG. 5B and FIG. 5C). It has been suggested that cellular IRESs may have evolved to support low level of expression in normal conditions and an inducible expression in response to different stimuli which can contribute to the development of several pathological condition in human like diabetes, cardiovascular disease and the development and progression of cancer (Komar *et al.,* 2005). bFGF IRES is specifically activated in the aorta wall in streptozotocin-induced diabetic mice, in correlation with increased expression of endogenous bFGF, which is one of the key of diabetes-linked atherosclerosis aggravation (Gonzalez-Herrera *et al.,* 2006). Angiotensin II plays a central role not only in the etiology of hypertension but also in the pathophysiology of cardiac hypertrophy, heart failure, vascular thickening, atherosclerosis and glomerulosclerosis in humans. The biological responses of Angiotention II are mediated by its interaction with angiotensin II type 1 receptor (AT1R), which is closely involved in the pathogenesis of cardiovascular disease. It was demonstrated that AT1R harbors an IRES, and activation of ATR1 play a pivotal role in the pathogenic process (Martin *et al.,* 2003).

It is well known that solid tumors grow in vivo as multicellular masses in which a proportion of cells is deprived of normal contacts with the basement membrane and is anoikisresistant. Cell lines derived from such solid tumors are capable of growing in an anchorage-independent manner as colonies in soft agar or suspension culture (Freedman *et al.,* 1974). The acquisition of anchorage-independence is an important hallmark of cancer cells and is thought to be one of the critical factors in the growth and metastasis of cancer. Although certain signaling pathways to abrogate the requirement for intergrin-extracellular matrix-mediated signaling function for anchorage-independent growth of cancer cells has been proposed, the precise molecular mechanism is not fully understood (Grossmann, 2002; Whang, 2004). Contrasted to its ineffectiveness in suppressing the proliferation of U-87 MG cells (FIG. 3A), thalidomide efficiently inhibited the anchorage-independent growth and aggregation of these cells at low dose (FIGs. 3B - 3E). Therefore, a novel tumor-suppressing mechanism of thalidomide it is realized. In this respect, positive correlations between the expression levels of bFGF and anchorage-independent growth of human fibroblast, prostatic epithelial cells and melanocytes have been reported. (Bikfalvi *et al.,* 1995; Quarto *et al.,* 1991; Nesbit *et al.,* 1999; Ropiquet *et al.,* 1997). Hence, down-regulation of colony formation in soft agar of U-87 MG cells by thalidomide attributed to a decreased bFGF expression it induced. This speculation was supported by the shRNA-mediated bFGF knock-down study which clearly showed a positive correlation between cellular bFGF levels and colony forming ability of these cells in soft agar (FIG. 7A, FIG. 7C and FIG. 7D). On the other hand, since the addition of recombinant human bFGF only partially rescued the loss of soft agar colony forming ability of U-87 MG cells (Table 3), the contribution of an intracrine signaling of this growth factor to cell transformation was postulated.

Even though the precise role of nuclear bFGF in U-87 MG cells is unclear, a stimulation of fibroblast growth in low serum by nuclear bFGF has been reported (Arese *et al.,* 1999). Moreover, the nuclear accumulation of bFGF in human astrocytic tumors has been shown as a useful predictor of patients' survival (Fukui *et al.,* 2003). Recent reports showed that cell-cell adhesion was important for anchorage-independent growth but might inhibit anchorage-dependent growth (Hokari *et al..* 2007). On the other hand, bFGF could regulate the expression of some adhesion molecules such as integrin in endothelial cells (Klein *et al.*, 1993) and glioma periphery (Bello *et al.,* 2001), which contain the G-rich promoter regions and may involve in anchorage-independent growth of embryonic developing tissue (Stephens *et al.,* 2000) and cancer cells (Bikfalvi *et al.,* 1995). Based on the present application, it is realized that therefore offers an evolutional insight into a new strategy for the development of novel anticancer drugs based on the mechanisms of anchorage-independence instead of conventional anchorage-dependent, and effectively to suppression of tumorigenicity involving growth and metastasis.

Many studies focus on the immunomodulatory activities of for it could potentially inhibit LPS induced TNF-α secretion by monocytes lower as at 0.3 µg/ml (Sampaio *et al.,* 1991), which is very close the effective concentration throughout the present application. It is said that thalidomide could down-regulate the activity of NF-κB, which is a transcription factor controls huge downstream pathways such as immune response and adhesion molecular expression (Li *et al.,* 2002), through inhibition of IκB kinase activity (Keifer *et al.,* 2001). However, it has been shown that bFGF could regulate IκB kinase activity by binding to the FGFR2 and activating of the downstream signaling pathway (Tang *et al.,* 2007). Beside this, bFGF had also been proved enhancing monocyte and neutrophil recruitment to inflammation, which might result in the amplification of the immunological signaling (Zittermaun *et al*., 2006). Therefore, the present application also highlights a unified molecular mechanism of thalidomide on down-regulation of bFGF expression and signaling, and consequently controls the downstream immune response for its immunomodulatory activity.

As mentioned above, the present application showed that the G- and/or GC-rich Sequence contained in the promoter and the transcripts of bFGF is the target for thalidomide to interact with, which causes the down-regulation of cellular bFGF expression level. The decrease of bFGF would lead to a down-shift of U-87 MG tumorigenicity mediated by anchorage-independent growth, which was confirmed by down-regulate the bFGF level by RNAi. The clinical daily application dose of thalidomide is between 200 to 800 mg in multiple myeloma and to a maximum of 1200 mg in glioma and renal cancer, and the administration would give the serum concentration about 1.8 to 10 µg/ml (Eleutherakis-Papaiakovou *et al.,* 2004). According to the present application, however, the effective concentration to inhibit the anchorage independent growth in U-87 MG cells, which is a kind of tumor with high bFGF basal level, was below the therapeutic one. Thus the dose needed for patients with higher bFGF serum level should be much lower than it is applied now, which might reduce the side effects. Besides this, it is realized that using some drug delivery system such as liposome enhances the bioactivity of thalidomide and reduces the side effects thereof. bFGF is not only one of the potent pro-angiogenic factors to endothelial cells, and it also acts as an upstream regulator to control the initiation of angiogenesis (Tsunoda *et al.,* 2007; Seghezzi *et al.,* 1998). Thus the activity of thalidomide in cancer patients might not only for it down-regulate the growth of tumor cells with high pre-treat bFGF expression level, but also for it suppressed the bFGF regulated angiogenesis.

Combining with the previous finding that bFGF could regulate the NF-κB signaling and functioned to amplify the inflammatory response by enhancing monocyte and neutrophil recruitment, a model for how thalidomide regulates angiogenesis, tumor growth and immune response was showed in FIG. 9, which offers a reasonable molecular mechanism of thalidomide based on the primary effect on the G- and/or GC-rich promoter and G- and/or GC-rich coding sequence of bFGF. The first level is on the G- and/or GC-rich promoter region of bFGF gene. A drug such as may bind to the G- and/or GC-rich promoter region of bFGF gene and thus down regulate the activity of the promoter. The second level is on the IRES of bFGF mRNA. A drug like thalidomide may bind to the IRES and thus down regulate the translation of bFGF transcript. A decrease in bFGF protein levels would lower tumorigenecity and down regulate bFGF-induced angiogenecis. In addition, a decrease in bFGF protein level might also diminish FGFR2-mediated signaling pathway, which would negatively impact nuclear NF-κ B site activity and result in a decrease in cellular immune response.

Based on the embodiments, it is realized that thalidomide down-regulates the expression of bFGF RNA transcripts by targeting its G- and/or GC-rich promoter at the relatively low concentration. A preferred embodiment also shows that down-regulates the expression of different bFGF isoforms in a dose-dependent manner, which is resulting from the change of the G-and/or GC-rich IRES activity. had been reported to be highly susceptible to hydrolysis in solution (Eriksson *et al.,* 1998), and the present application further provides a method for increasing the bio-availability of thalidomide by a slow-release technology, such as encapsulated by liposome and TMC. Since the embodiments of the present invention show that the bio-availability of would be increased by a relative slow-release technology, those applications based on the present invention and the relevant technologies disclosed in the literatures (such as Gomez-Orellana I. 2005; Lambkin I. *et al.* 2002; Lamprecht A, 2004; Li C.L. 2005; Mustata G. *et al.* 2006; Ranade VV. 1991; Rogers JA. *et al.* 1998; Taira MC. *et al.* 2004; Tiwari SB. *et al.* 2008; Zheng AP. *et al.* 2006) should all be under the spirits of the present invention.

All of the references cited herein are incorporated by reference in their entirety.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments and examples were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its spirit and scope. Accordingly, the scope of the present invention is defined by the appended claims rather than the foregoing description and the exemplary embodiments described therein.

### LIST OF REFERENCES

Aguayo A. et al. 2000. Blood. 96:2240-2245.
Andrys C. et al. 2007. Archives of Dermatological Research. 298:479-483.
Arese M et al. 1999. Mol. Biol. Cell 10:1429-1444.
Bakay B et al. 1968. J. Pharmacol. Exp. Ther. 161:348-360.
Bello L et al. 2001. Neurosurgery 49:380-389.
Benisty JI et al. 2004. Chest. 126:1255-1261.
Bethea JR et al. 1997. J Immunol. 158:5815-5823.
Bikfalvi A et al. 1995. J Cell Biol. 129:233-243.
Bikfalvi A et al. 1997. Endocr. Rev. 18:26-45.
Bonnal. S et al 2003. J Biol Chem. 278:39330-39336.
Bosco AA et al. 2003. Diabetologia. 46:1669-1675.
Boston M et al. 1997. Br J Ophthalmol 81:228-33.
Creancier L et al. 2000, J. Cell Biol 150:275-281.
Di Sabatino A et al. 2004. Inflamm Bowel Dis. 10:573-577.
Drucker L et al. 2003. Mol. Pharmacol. 64:415-420.
Eleutherakis-Papaiakovou V et al. 2004. Annals of Oncology. 15:1151-1160
Erdem F et al. 2005. Rheumatology International 25:599-603.
Eriksson T et al. 1998. Chirality 10:223-228.
Fang JY et al. 2005. J. Drug Target 13:19-27.
Fine HA et al 2000. J. Clin. Oncol. 18:708-715.
Florkeiwicz RZ et al. 1989. Proc. Natl Acad. Sci. U. S. A 86:3978-3981.
Frank RN. 1997. Ophthalmic Res. 29:341-353.
Franks ME et al. 2004. Lancet 363:1802-1811.
Freedman VH et al. 1974. Cell 3:355-359.
Fuhrmann-Benzakein E et al. 2000. Int. J. Cancer. 85:40-45.
Fukui S et al. 2003. Cancer 97:3061-3067.
Giles FJ et al. 2004. Leukemia Research. 28:595-604.
Gomez-Orellana I. 2005. Expert Opinion on Drug Delivery. 2:419-33
Gonzalez-Herrera IG et al. 2006. Biochem. Soc. Trans. 34:17-21.
Grossmann J. 2002. Apoptosis. 7:247-260.
Gullestad L et al. 2005. Circulation. 112:3408-3414.
Hokari M et al. 2007. Life Sci. 81:336-345.
Hoshino M et al. 2001. J Allergy Clin Immunol. 107:295-301.
Huang PH et al. 1990. Teratog. Carcinog. Mutagen. 10:281-294.
Huang PH et al. 1999. Pharmacol. Toxicol. 85:103-104*.*
Hurley LH et al. 2000. Pharmacol. Ther. 85:141-158.
Iwasaki A et al. 2004. European Journal of Cardio-thoracic Surgery. 25:443-448.
Kakimoto T et al. 2002. Jpn. J. Cancer Res. 93:1029-1036.
Ke LD et al. 2000. Clin. Cancer Res. 6:2562-2572.
Keifer JA et al. 2001. J Biol. Chem. 276:22382-22387.
Kelm JM et al. 2003. Biotechnol Bioeng, 83:173-180.
Kikuchi K et al. 2005. J Am Acad Dermatol. 53:57-61.
Klein S et al. 1993. Mol. Biol. Cell 4:973-982.
Komar AA et al. 2005. J. Biol. Chem. 280:23425-23428.
Kumar S et al. 2004. J. Clin. Oncol. 22:2477-2488.
Lambkin I. et a. 2002l. Expert Opinion on Biological Therapy. 2:67-73.
Lamprecht A et al. 2004. European Journal of Pharmaceutics & Biopharmaceutics. 58:37-43.
Lander ES, et al. 2001. Nature. 409:860-921.
Lawrence A et al. 2006. Dermatol Online J. 12:2.
Leonardi A et al. 2000. invest Ophlhalmol Vis Sci. 41:4175-4181.
Li C.L. 2005. Journal of Pharmacy & Pharmacology. 57:533-46.
Li X, Stark GR. 2002. Exp. Hematol. 30:285-296.
Martin MM et al. 2003. Mol. Cell Endocrinol. 212:51-61.
Matthews SJ et al. 2005. Clin. Ther. 25:342-395.
Mi FL et al. 2008. Bioconjug Chem. 19:1248-1255.
Mignatti P et al. 1992. J Cell Physiol 151:81-93.
Mukdsi 3H et al. 2006. Acta Neuropathol. (Berl) 112:491-501.
Murphy PR et al. 1992. Mol. Endocrinol 6:877-884.
Mustata G et al. 2006. Critical Reviews in Therapeutic Drug Carrier Systems. 23:111-35
Nakashima M et al. 1994. Annals of the Rheumatic Diseases. 53:45-50.
Neben K et al. 2001. Clin. Cancer Res. 7:2675-2681.
Nesbit M et al. 1999. Oncogene 18:6469-6476.
Nguyen M et al. 1994. J Natl Cancer Inst 86:356-361.
Nicholls PJ. 1966. J. Pharm. Pharmacol. 18:46-48.
Nociari MM et al. 1998. J. Immunol. Methods 213:157-167.
Okmnura M et al. 1996. Arzneimittel forschung. 46:727-39.
Poon RT et al. 2001. J Clin Oncol. 19:1207-1225.
Qin Y et al. 2008. Biochimie. 90:1149-1171.
Quarto N et al. 1991. Cell Regul. 2:699-708.
Quarto N et al. 2005. Gene 356:49-68.
Ranade VV. 1991. Journal of Clinical Pharmacology. 31:98-115.
Renko M et al.. 1990. J Cell Physiol 144:108-114.
Richardson P et al. 2002. Annu. Rev. Med. 53:629-657.
Rogers JA. et al. 1998. Critical Reviews in Therapeutic Drug Carrier Systems. 15:421-80.
Ropiquet F et al. 1997. Int. J. Cancer 72:543-547.
Samaniego F et al. 1998. Am J Patbol. 152:1433-1443*.*
Sampaio EP et al. 1991. J. Exp. Med. 173:699-703.
Sato N et al. 2002. Jpn. J. Cancer Res. 93:459-466.
Scalapino KJ et al.2003. Clin Exp Med. 2:159-165.
Seghezzi G et al.. 1998. J. Cell Biol. 141:1659-1673.
Sezer O et al. 2001. Eur J Haematol. 66:83-88.
Singhal S et al. 1999. N. Engl. J. Med. 341:1565-1571.
Stephens TD et al. 2000. Biochem. Pharmacol. 59:1489-1499.
Taira MC. et al. 2004. Drug Delivery 11:123-8.
Tang CH et al. 2007. J Cell Physiol 211:45-55.
Teo SK et al. 2005. Drug Discovery Today. 10:107-114.
Thanou MM et al. 2000. J Control Release. 64:15-25.
Tiwari SB. et al. 2008. Methods in Molecular Biology. 437:217-43.
Tsunoda S et al. 2007. Cancer Sci. 98:541-548.
Ueno K et al. 2001. Lung Cancer. 31:213-219.
Ugurel S et al. 2001. J Clin Oncol. 19:577-583.
Usha S et al. 2005. J. Biochem. Mol. Biol. 38:198-205.
Vacca A et al. 2005. J. Clin. Oncol. 23:5334-5346,
Vagner S et al. 1995. Mol. Cell Biol. 15:35-44.
Vagner S et al. 1996. J Cell Biol. 135:1391-1402.
Venter JC, et al. 2001. Science 291, 1304.
Whang LH. 2004. Mi. Sinai J. Med. 71:361-367.
Wu JJ et al. 2005. British Journal of Dermatology. 153:254-273.
Yamanaka Y et al. 1993. Cancer Res. 53:5289-5296.
Zheng AP. et al. 2006. Journal of Nanoscience & Nanotechnology. 6:2936-44.
Zittermann SI et al. 2006. Am. J. Pathol. 168:835-846.

## Claims

1. Thalidomide for use in treating a bFGF overexpression-associated disease selected from the group consisting of cancer, immunological disorder, angiogenesis-associated disease and sleep disorder, **characterized in that** it is formulated as a slow-release pharmaceutical composition adapted to release thalidomide in an amount effective to down-regulate the expression of bFGF RNA transcripts by targeting its G- and/or GC-rich promoter.

2. Thalidomide for use according to claim 1, wherein the cancer is one selected from the group consisting of brain tumor, prostate cancer, pancreatic cancer, breast cancer, lung cancer, head and neck cancer, renal cell carcinoma, colorectal carcinoma, hepatocellular carcinoma, ovarian carcinoma, endometrial carcinoma, bladder cancer, prolactinoma, melanoma, Kaposis's sarcoma, soft tissue sarcoma, multiple myeloma, myelodysplastic syndrome, non-Hodgkin's lymphoma and leukemia.

3. Thalidomide for use according to claim 1, wherein the immunological disorder is one selected from the group consisting of rheumatoid arthritis, osteoarthritis, Behcet's disease, systemic sclerosis, polyarteritis nodosa, psoriasis, asthma, vernal keratoconjunctivitis and Crohn's disease.

4. Thalidomide for use according to claim 1, wherein the angiogenesis-associated disease is one selected from the group consisting of pulmonary arterial hypertension, rheumatoid arthritis, asthma, psoriasis, proliferative diabetic retinopathy and age-related macular degeneration.


SEQUENCE LISTING

<110> Wu, Rong-Tsun
<120> METHODS AND PHARMACEUTICAL COMPOSITIONS FOR REGULATION OF G- AND/OR
GC-RICH NUCLEIC ACID EXPRESSION

<130> 10004-012PCT

<150> US 60/989,831
<151> 2007-11-22

<160> 19

<170> checker 4.4

<210> 1
<211> 21
<212> DNA
<213> Artificial Sequence

<220>
<221> glyceraldehyde 3-phosphate dehydrogenase-forward (GAPDH-F)
<223> primer

<400> 1
aatgtcaccg ttgtccagtt g
21

<210> 2
<211> 19
<212> DNA
<213> Artificial Sequence

<220>
<221> glyceraldehyde 3-phosphate dehydrogenase-reverse (GAPDH-R)
<223> primer

<400> 2
gtggctgggg ctctacttc
19

<210> 3
<211> 22
<212> DNA
<213> Artificial Sequence

<220>
<221> basic fibroblast growth factor-forward (bFGF-F)
<223> primer

<400> 3
atcaaaggag tgtgtgctaa cc
22

<210> 4
<211> 21
<212> DNA
<213> Artificial Sequence

<220>
<221> basic fibroblast growth factor-reverse (bFGF-R)
<223> primer

<400> 4
actgcccagt tcgtttcagt g
21



<210> 5
<211> 23
<212> DNA
<213> Artificial Sequence

<220>
<221> basic fibroblast growth factor promoter-forward (bFGF-P-F)
<223> primer

<400> 5
gtggcacctg ctatatccta ctg
23



<210> 6
<211> 17
<212> DNA
<213> Artificial Sequence

<220>
<221> basic fibroblast growth factor promoter-reverse (bFGF-P-R)
<223> primer

<400> 6
agcctcgagc cgctcgg
17



<210> 7
<211> 1070
<212> DNA
<213> Artificial Sequence

<220>
<221> basic fibroblast growth factor promoter (bFGF-promoter)
<223> promoter

<400> 7
gtggcacctg ctatatccta ctgaaaatta ccaaaatgca attaacttca attttacatt
60
tgggatttac agaaaataac tctctctcca agaaatgcat aacaatttag ctagggcaaa
120
tgccaggtcc gagttaagac attaatgcgc ttcgatcgcg ataaggattt atccttatcc
180
ccatcctcat ctttctgcgt cgtctaattc aagataggtc agtaaaggaa accttttcgt
240
tttagcaacc caatctgctc cccttctctg gcctctttct ctccttttgt tggtagacga
300
cttcagcctc tgtcctttaa ttttaaagtt tatgccccac ttgtacccct cgtcttttgg
360
tgatttagag attttcaaag cctgctctga cacagactct tccttggatt gcaacttctc
420
tactttgggg tggaaacggc ttctccgttt tgaaacgcta gcggggaaaa aatgggggag
480

aaagttgagt ttaaactttt aaaagttgag tcacggctgg ttgcgcagca aaagccccgc
540
agtgtggaga aagcctaaac gtggtttggg tggtgcgggg gttgggcggg ggtgactttt
600
gggggataag gggcggtgga gcccagggaa tgccaaagcc ctgccgcggc ctccgacgcg
660
cgccccccgc ccctcgcctc tcccccgccc ccgactgagg ccgggctccc cgccggactg
720
atgtcgcgcg cttgcgtgtt gtggccgaac cgccgaactc agaggccggc cccagaaaac
780
ccgagcgagt agggggcggc gcgcaggagg gaggagaact gggggcgcgg gaggctggtg
840
ggtgtggggg gtggagatgt agaagatgtg acgccgcggc ccggcgggtg ccagattagc
900
ggacgcggtg cccgcggttg caacgggatc ccgggcgctg cagcttggga ggcggctctc
960
cccaggcggc gtccgcggag acacccatcc gtgaacccca ggtcccgggc cgccggctcg
1020
ccgcgcacca ggggccggcg gacagaagag cggccgagcg gctcgaggct
1070

<210> 8
<211> 20
<212> DNA
<213> Artificial Sequence

<220>
<221> enhanced green fluorescence protein-forward (EGFP-F)
<223> primer

<400> 8
ccatggtgag caagggcgag
20

<210> 9
<211> 21
<212> DNA
<213> Artificial Sequence

<220>
<221> enhanced green fluorescence protein-reverse (EGFP-R)
<223> primer

<400> 9
tcagggtcag cttgccgtag g
21

<210> 10
<211> 30
<212> DNA
<213> Artificial Sequence

<220>
<221> low molecular weight-internal ribosome entry site-forward (LMW-IRES-F)
<223> primer

<400> 10
ctcctgacgc ggggccgtgc cccggagcgg
30

<210> 11
<211> 31
<212> DNA
<213> Artificial Sequence

<220>
<221> low molecular weight-internal ribosome entry site-reverse (LMW-IRES-R)
<223> primer

<400> 11
ctcacaacgg gttgtgaggg tcgctcttct c
31

<210> 12
<211> 307
<212> DNA
<213> Artificial Sequence

<220>
<221> low molecular weight-internal ribosome entry site-sequence (LMW-IRES-S)
<223> RBS

<400> 12
ctcctgacgc ggggccgtgc cccggagcgg gtcggaggcc ggggccgggg ccgggggacg
60
gcggctcccc gcgcggctcc agcggctcgg ggatcccggc cgggccccgc agggaccatg
120
gcagccggga gcatcaccac gctgcccgcc ttgcccgagg atggcggcag cggcgccttc
180
ccgcccggcc acttcaagga ccccaagcgg ctgtactgca aaaacggggg cttcttcctg
240
cgcatccacc ccgacggccg agttgacggg gtccgggaga agagcgaccc tcacaacccg
300
ttgtgag
307


<210> 13
<211> 31
<212> DNA
<213> Artificial Sequence

<220>
<221> high molecular weight-internal ribosome entry site-forward (HMW-IRES-F)
<223> primer

<400> 13
ctcctgacgc gtcaggaggg aggaggactg g
31


<210> 14
<211> 31
<212> DNA
<213> Artificial Sequence

<220>
<221> high molecular weight-internal ribosome entry site-reverse (HMW-IRES-R)
<223> primer

<400> 14
ctcacaacgg gttgtgaggg tcgctcttct c

31

<210> 15
<211> 632
<212> DNA
<213> Artificial Sequence

<220>
<221> high molecular weight-internal ribosome entry site-sequence
(HMW-IRES-S)
<223> RBS

<400> 15
ctcctgacgc gtcaggaggg aggagaactg ggggcgcggg aggctggtgg gtgtgggggg
60
tggagatgta gaagatgtga cgccgcggcc cggcgggtgc cagattagcg gacgcggtgc
120
ccgcggttgc aacgggatcc cgggcgctgc agcttgggag gcggctctcc ccaggcggcg
180
tccgcggaga cacccatccg tgaaccccag gtcccgggcc gccggctcgc cgcgcaccag
240
gggccggcgg acagaagagc ggccgagcgg ctcgaggctg ggggaccgcg ggcgcggccg
300
cgcgctgccg ggcgggaggc tggggggccg gggccggggc cgtgccccgg agcgggtcgg
360
aggccggggc cggggccggg ggacggcggc tccccgcgcg gctccagcgg ctcggggatc
420
ccggccgggc cccgcaggga ccatggcagc cgggagcatc accacgctgc ccgccttgcc
480
cgaggatggc ggcagcggcg ccttcccgcc cggccacttc aaggacccca agcggctgta
540
ctgcaaaaac gggggcttct tcctgcgcat ccaccccgac ggccgagttg acggggtccg
600
ggagaagagc gaccctcaca acccgttgtg ag
632


<210> 16
<211> 21
<212> DNA
<213> Artificial Sequence

<220>
<221> shRNA No.1
<223> primer

<400> 16
gcagtcataa acagaagaat a
21


<210> 17
<211> 21
<212> DNA
<213> Artificial Sequence

<220>
<221> shRNA No.2
<223> primer

<400> 17
gaccctcaca tcaagctaca a
21

<210> 18
<211> 21
<212> DNA
<213> Artificial Sequence

<220>
<221> shRNA No.3
<223> primer

<400> 18
ctatcaaagg agtgtgtgct a
21


<210> 19
<211> 22
<212> DNA
<213> Artificial Sequence

<220>
<221> sh green fluorescence protein control (shGFP control)
<223> primer

<400> 19
acgtctatat caatggccga ca
22